(19) Europäisches Patentamt
European Patent Office
Office européen des brevets

(11) **EP 3 545 832 B1**

(12) **EUROPEAN PATENT SPECIFICATION**

(45) Date of publication and mention
of the grant of the patent:
**20.03.2024   Bulletin 2024/12**

(21) Application number: **17874481.9**

(22) Date of filing: **20.11.2017**

(51) International Patent Classification (IPC):
***A61B 5/0285*** *(2006.01)*      ***A61B 5/021*** *(2006.01)*
***A61B 5/00*** *(2006.01)*

(52) Cooperative Patent Classification (CPC):
**A61B 5/0285; A61B 5/02125;** A61B 5/01;
A61B 5/02116

(86) International application number:
**PCT/CN2017/111799**

(87) International publication number:
**WO 2018/095291 (31.05.2018 Gazette 2018/22)**

(54) **CORRECTION METHOD FOR PULSE WAVE PROPAGATION TIME RELATED TO DIASTOLIC BLOOD PRESSURE AND SYSTOLIC BLOOD PRESSURE**

KORREKTURVERFAHREN FÜR PULSWELLENAUSBREITUNG IM ZUSAMMENHANG MIT DIASTOLISCHEM BLUTDRUCK UND SYSTOLISCHEM BLUTDRUCK

PROCÉDÉ DE CORRECTION DE LA DURÉE DE PROPAGATION D'ONDES PULSATILES EN RAPPORT AVEC LA PRESSION ARTÉRIELLE DIASTOLIQUE ET LA PRESSION ARTÉRIELLE SYSTOLIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.11.2016   CN 201611045054**
**22.11.2016   CN 201611046184**

(43) Date of publication of application:
**02.10.2019   Bulletin 2019/40**

(73) Proprietor: **Zhejiang Mailian Medical Equipment
Co., Ltd
Hangzhou, Zhejiang 311100 (CN)**

(72) Inventors:
• **CHEN, Yan
Chongqing 400000 (CN)**
• **CHEN, Yu
Chongqing 400000 (CN)**

(74) Representative: **Cabinet Chaillot
16/20, avenue de l'Agent Sarre
B.P. 74
92703 Colombes Cedex (FR)**

(56) References cited:
**EP-A1- 0 443 267       CN-A- 102 008 296
CN-A- 102 258 364       CN-A- 103 385 702
CN-A- 104 382 571       CN-A- 106 377 238
CN-A- 106 580 303**

• **Chen Yan: "Continuous and Noninvasive Blood
Pressure Measurement by Pulse Wave Velocity:
a New and Systematic Modeling Methodology", ,
9 November 2012 (2012-11-09), pages 1-216,
XP055701733, Retrieved from the Internet:
URL:https://dr.ntu.edu.sg/bitstream/10356/
50792/1/TeG0702515F.pdf [retrieved on
2020-06-05]**
• **YAN CHEN ET AL: "Continuous and Noninvasive
Measurement of Systolic and Diastolic Blood
Pressure by One Mathematical Model with the
Same Model Parameters and Two Separate Pulse
Wave Velocities", ANNALS OF BIOMEDICAL
ENGINEERING, KLUWER ACADEMIC
PUBLISHERS-PLENUM PUBLISHERS, NE, vol.
40, no. 4, 19 November 2011 (2011-11-19), pages
871-882, XP035033704, ISSN: 1573-9686, DOI:
10.1007/S10439-011-0467-2**

**Description**

BACKGROUND OF THE INVENTION

1. Field of the Invention

The present invention relates to the technical field of arterial blood pressure measurement, and specifically relates to a method for correcting pulse wave transit time associated with diastolic blood pressure or systolic blood pressure.

2. Description of Related Art

Arterial blood pressure is one of the main indicators for reflecting the state of a circulatory system and assessing organ perfusion, and is an important vital sign parameter for perioperative monitoring. At present, the methods of perioperative blood pressure monitoring can be divided into invasive measurement and non-invasive measurement. Invasive measurement refers to a technique of placing a catheter into artery, and converting Intra-arterial pressure into an electronic signal through a transducer, and display the blood pressure signal in real time on a monitoring device. Invasive method can measure beat-to-beat blood pressure continuously and accurately, but the possible dangers and injuries cannot be ignored. Oscillometric method is commonly used for non-invasive measurement, which is simple to operate, is clinically recognized in accuracy and is widely used for health checkup and perioperative monitoring. However, the oscillometric method only measures blood pressure intermittently in every 3-5 minutes intraoperatively, and cannot track changes in arterial blood pressure in real time.

To this end, some techniques for continuous and non-invasive measurement of beat-to-beat blood pressure have been proposed, in which the measurement method based on pulse wave transit time/velocity (PTT/PWV) has gradually become a research hotspot. The method is to simultaneously acquire photoplethysmography (PPG) signals and an electrocardiogram (ECG) by one or more photosensors and electrocardiographic electrodes, and PTT/PWV is calculated by using the time difference between a PPG and an ECG or the time difference between two PPGs; Afunctional relationship or mathematical model between PTT/PWV and blood pressure is established, so that the measurable PTT/PWV can be used for calculating blood pressure. Some academic papers have reported the principle of continuous and non-invasive measurement of beat-to-beat blood pressure using PTT/PWV, for example, Yan Chen, Changyun Wen, Guocai Tao, Min Bi, and Guoqi Li A *Novel Modeling Methodology of the Relationship Between Blood Pressure and Pulse Wave Velocity*; Yan Chen, Changyun Wen, Guocai Tao and Min Bi *Continuous and Non-invasive Measurement of Systolic and Diastolic Blood Pressure by One Mathematical Model with the Same Model Parameters and Two Separate Pulse Wave Velocities*; Younhee Choi, Qiao Zhang, Seokbum Ko *Non-invasive cuffless blood pressure estimation using pulse transit time and Hilbert Huang transform*; Zheng Y, Poon CC, Yan BP, Lau JY *Pulse Arrival Time Based Cuff-Less and 24-H Wearable Blood Pressure Monitoring and its Diagnostic Value in Hypertension*; Mukkamala R, Hahn JO, Inan OT, Mestha LK, Kim CS, Töreyin H, Kyal S *Toward Ubiquitous Blood Pressure Monitoring via Pulse Transit Time: Theory and Practice.* Moreover, some patents disclose specific methods or devices for continuous and non-invasive measurement of beat-to-beat blood pressure using PTT/PWV, for example, Chinese patents CN101229058A, CN102811659A and CN1127939C, U.S. Patents 5865755, 5857975, 5649543 and 9364158, and European Patent 0413267, etc.

Existing methods and techniques for measuring blood pressure using PTT/PWV require conventional auscultatory or oscillometric method to measure one or more blood pressure values for initial calibration. The reason for the calibration is that the relationship between PTT/PWV and blood pressure is object-dependent, that is, there is a definite relationship between PTT/PWV and blood pressure of each individual. The purpose of calibration is to determine mathematical model parameters that are adaptive to the object.

However, the existing methods have certain limitations and can only be applied under the condition that the circulatory system is not subjected to external interference, because only in the absence of interference, the relationship between PTT and blood pressure has strong regularity for individuals, and can be described by certain functions and mathematical models. However, during the perioperative period, the circulatory system of a patient is influenced by fluid therapy, drugs, surgical operations, temperature, etc., which leads to a series of irregular changes of PTT. Use of an irregular PTT and an intrinsic mathematical model to calculate blood pressure may produce large errors. Because the relationship between irregular PTT and blood pressure no longer has certain regularity, even if the mathematical model parameters are frequently calibrated to adapt to the PTT irregularities, the fundamental problem is not solved, and consequently, the accuracy and realtime performance of existing methods cannot meet the requirement of clinical blood pressure measurement.

SUMMARY OF THE INVENTION

In view of deficiencies in the prior art, the present invention is directed to provide, a method according to claim 1.

DESCRIPTION OF THE EMBODIMENTS

The embodiments of the technical solution of the present invention will be described in detail below. The following embodiments are only used for more clearly illustrating the technical solutions of the present invention, and thus are only examples, and not intended to limit the protection scope of the present invention.

Pulse wave transit time (PTT) changes in perioperative period can be divided into two categories: type I changes: PTT changes caused by changes in blood pressure; and type II changes: unsynchronized changes in PTT and blood pressure (the direction or amount of changes of the two does not conform to a regular function rule). For example, when the blood volume is mildly insufficient, PTT will increase, but due to the adjustment on peripheral resistance of the body, blood pressure may not change much. The use of a hook in thoracoabdominal surgery may seriously affect PTT, but has less effect on blood pressure. Norepinephrine makes small arteries strongly contract and the blood pressure significantly increase, but the effect on the average PTT of the whole body is small.

When PTT has type I changes, the relationship between PTT and blood pressure can still be expressed by a certain function, and the change in blood pressure can be estimated by a mathematical model. While when PTT has type II changes, using a mathematical model based on a conventional circulatory system to estimate blood pressure will produce large errors. The errors are principle errors in measurement of blood pressure by using PTT and cannot be solved by initial calibration and periodic calibration of mathematical model parameters. The difference in PTT among different individuals and the irregular change of PTT in the same individual are two different types of problems, which need to be solved by different methods. To this end, the present invention extracts various variables based on the features of the pulse wave to identify and adaptively correct various type II changes of the PTT, and overcome the principle errors; the available mathematical models can be combined to form a continuous and non-invasive measurement method of blood pressure with an adaptive calibration function, without the need to rely on conventional methods such as oscillometry for repeated calibration.

Positions of the human body for detecting the pulse wave are preferably the ear and the toe. The pulse waves of these two parts contain the physiological and pathological information of the aorta and peripheral arteries, and are representative in propagation paths. A sensor for detecting the pulse signal is preferably an infrared photoplethysmograph (PPG).

The feature changes of ear and toe pulse waves, and the relative changes in features between the two pulse waves provide rich information for identifying the type II changes in PTT and the changes in blood pressure difference between different sites of the body. The present invention collects the invasive arterial blood pressure, pulse waves of the ears and toes, and PTT of a large number of surgical cases for several years for analyzing, extracts various variables according to the feature changes and relative feature changes of the two pulse waves, studies the relationship between different variables and different type II changes of PTT, and defines the application scopes of these variables.

In clinical application, during continuous measurement of blood pressure using PPT, the pulse waveform is analyzed in real time and variables are extracted. Whether the PTT has the type II changes is determined according to whether the variables fall within the application scope, and the nature and extent of the type II changes of the PTT are determined according to the nature of the applicable variables. If a variable is outside the application scope, the corresponding type II changes do not occur in the PTT, then the variable is not applicable. Several applicable variables are fused to calculate the correction amount to correct the PTT. The corrected PTT/PWV is applicable to the available mathematical models to accurately calculate the blood pressure.

The present invention uses limited variables to express the most important changes of pulse wave form, and studies the relationship between these changes and PTT. As for the pulse wave in plane coordinates, the ordinate is amplitude h, the abscissa is time t, and the pulse wave starting point is the coordinate origin.

Embodiment 1:

A method for correcting PTT associated with diastolic blood pressure, includes the following steps:

S1) detecting a pulse wave at an ear in each cardiac cycle in real time and obtaining the following data: the height of an aortic valve closure point on an ear pulse wave denoted as $h_{sd}$, that is, the height at a junction between the systolic and diastolic phases on the ear pulse wave, the systolic time of the ear pulse wave denoted as $t_s$, the diastolic time of the ear pulse wave denoted as $t_d$, and the maximum height of the ear pulse wave denoted as $h_{max}$;
S2) detecting the pulse wave at a toe in each cardiac cycle in real time and obtaining the following data: the systolic time of the toe pulse wave denoted as $t_{s\text{-}toe}$, the diastolic time of the toe pulse wave denoted as $t_{d\text{-}toe}$, the maximum

height of the toe pulse wave denoted as $h_{max\text{-}toe}$, the time interval between the starting point to the midpoint of the peak of the toe pulse wave denoted as $t_{ch\text{-}toe}$, and the time interval between the starting point to the peak of the toe pulse wave denoted as $t_{max\text{-}toe}$, where the midpoint of the peak refers to the midpoint of arising edge turning point and a falling edge turning point at the peak; the definition of the midpoint of the peak can be understood by referring to the literature YAN CHEN, CHANGYUN WEN, GUOCAI TAO, and MIN BI *Continuous and Non-invasive Measurement of Systolic and Diastolic Blood Pressure by One Mathematical Model with the Same Model Parameters and Two Separate Pulse Wave Velocities.*

S3) calculating the PTT associated with diastolic blood pressure denoted as $T_d$, and the definition can be understood by referring to the literature YAN CHEN, CHANGYUN WEN, GUOCAI TAO, and MIN BI *Continuous and Non-invasive Measurement of Systolic and Diastolic Blood Pressure by One Mathematical Model with the Same Model Parameters and Two Separate Pulse Wave Velocities*; $h$ is the amplitude of the ear pulse wave or the toe pulse wave in a longitudinal direction;

S4) by using the data in the same cardiac cycle acquired through step S1 and step S2, calculating a few correction variables in the cardiac cycle;

S5) according to the correction variables in the cardiac cycle acquired in step S4, calculating a correction matrix in the cardiac cycle; and

S6) continuously acquiring the correction matrices in a plurality of cardiac cycles, and correcting the $T_d$ acquired in the step S3.

By the method, the PTT associated with diastolic blood pressure is determined by a time difference of an ear pulse wave and a toe pulse wave in the same cardiac cycle, and a few correction variables are extracted based on the pulse wave features, then a correction matrix is acquired to perform adaptive correction on the irregular change of pulse wave transit time. The corrected transit time can be used with available mathematical models for continuously and accurately measuring diastolic blood pressure in each cardiac cycle in a clinical setting.

First correction variable $b_1$:

The correction variables obtained in the step S4 include a first correction variable $b_1$. $b_1$ is used for correcting the type II changes in $T_d$ in a hypotensive state, the applicable range of $b_1$ is $b_1 > 0$, and if $b_1$ is larger, the blood pressure

$$k_{sd-m-0} = \frac{t_s h_{sd}}{\int_0^{t_s} hdt}$$

is lower. , $k_{sd\text{-}m\text{-}0}$ represents the ratio of $h_{sd}$ to the average height of the ear pulse wave systole. In some cases, under a hypotensive state, the pulse wave peak appears as a forward-inclined triangle When $h_{sd}$ decreases a lot, $k_{sd\text{-}m\text{-}0}$ becomes smaller, indicating that the waveform at the end of the aortic systole is much lower, the continuous power for pushing pulse wave transit is insufficient, and the transit time is prolonged. In this state, the diastolic information is unstable and should not be used.

$d_{1\text{-}b} = 74$ to 82, preferably is 78. $d_{1\text{-}2\text{-}b} = 98$ to 106, preferably is 102.

When the continuous power for pushing pulse wave transit is insufficient, the transit time $T_d$ is prolonged and needed be corrected by $b_1$. That is, if $d_{1\text{-}b} \le k_{sd\text{-}m\text{-}0} \le d_{1\text{-}2\text{-}b}$, then $b_1 = (d_{1\text{-}2\text{-}b} - k_{sd\text{-}m\text{-}0}) \times 0.4$.

When the continuous power for pushing pulse wave transit is seriously insufficient, the transit time $T_d$ is prolonged a lot, and $b_1$ takes the upper limit value for correction. That is, if $k_{sd\text{-}m\text{-}0} < d_{1\text{-}b}$, then $b_1 = 24 \times 0.4$.

When the continuous power for pushing pulse wave transit is sufficient, $T_d$ does not need to be corrected, and $b_1$ is not applicable. That is, if $k_{sd\text{-}m\text{-}0} > d_{1\text{-}2\text{-}b}$, then set$b_1 = 0$.

Second correction variable $b_2$:

The correction variables obtained in the step S4 also include a second correction variable $b_2$, $b_2$ is used for correcting the type II changes in $T_d$ in a hypertensive state, the applicable range of $b_2$ is $b_2 > 0$, and if $b_2$ is larger, the

$$k_{sd-m-ts} = \frac{t_s h_{sd}}{\int_{t_s}^{2t_s} hdt}$$

diastolic blood pressure is higher. , $k_{sd\text{-}m\text{-}ts}$ represents the ratio of $h_{sd}$ to the average height of the $t_s$ to $2t_s$ segments of the ear pulse wave diastole, and is used for determining the irregular change of the pulse wave diastole. For example, in a thoracoabdominal surgery, an upward pulling hook causes the aortic stress to change, so

$$k_{sd-m-2} = \frac{2t_s h_{sd}}{\int_0^{2t_s} h\,dt}$$

that the amplitude of the ear pulse wave diastole is reduced, and the $k_{sd-m-ts}$ becomes larger.
$k_{sd-m-2}$ represents the ratio of $h_{sd}$ to the average height of the ear pulse wave 0 to $2t_s$ segments, includes the information of systolic and partial diastolic waveform, and is mainly used for a hypertensive state, such as increase of heart rate and blood pressure caused by tracheal intubation. In the state of hypertension, the ear pulse wave appears an equilateral triangle or a backward-inclined triangle, $h_{sd}$ rises a lot, and $k_{sd-m-2}$ becomes larger. Compared with the waveform in a normal blood pressure state, the slope of the rising edge of the waveform in the hypertensive state becomes smaller, the power for pushing pulse wave transit is insufficient, and the transit time $T_d$ is prolonged.

If

$$|k_{sd-m-0} - k_{sd-m-ts}| \geq 40 \text{ and } (k_{sd-m-0} + k_{sd-m-ts})/2 \geq k_{sd-m-2},$$

then

$$k_{sd-m} = 2 \times k_{sd-m-2} - (k_{sd-m-0} + k_{sd-m-ts})/2,$$

otherwise $k_{sd-m} = k_{sd-m-2}$;

If the waveform of the ear pulse wave diastole has an irregular change, for example, if the upward pulling hook of the thoracoabdominal surgery causes the aortic stress to change, and the form of the pulse wave diastole changes significantly, $k_{sd-m}$ is corrected, otherwise $k_{sd-m} = k_{sd-m-2}$.

$d_{2-b} = 1.33$ to $1.43$, preferably is $1.38$.

If $k_{sd-m} > (d_{2-b} + (age-14)/15/100)$, where $age$ is age, the continuous power corresponding to the diastolic pressure is insufficient, the transit time $T_d$ is relatively prolonged, and needed be corrected by $b_2$, then $b_2 = (k_{sd-m} - (d_{2-b} + (age-14)l15/100)) \times 0.5$, the change of $b_2$ is inversely proportional to the change of the slope of the pulse wave rising edge, where $0.5$ is the proportional coefficient.

If $k_{sd-m} \leq (d_{2-b} + (age-14)l15/100)$, the continuous power corresponding to the diastolic pressure is sufficient, $b_2$ is not applicable, then set $b_2 = 0$.

Third correction variable $b_3$:

The correction variables obtained in the step S4 further include a third correction variable $b_3$, which is used for correcting the $T_d$ in a state that the blood volume changes or the body temperature of a sensor placement site changes.

$$k_{d-m-t_d} = \frac{\int_{t_s}^{t_s+t_d} h\,dt}{t_d h_{max}}$$

, $k_{d-m-t_d}$ is the ratio of the average height of the ear pulse wave diastole to the maximum height $h_{max}$. The blood volume is reduced when a patient fasts and drinks less water before surgery, $k_{d-m-t_d}$ decreases, and the transit time is prolonged; when the blood volume increases due to blood transfusion and IV transfusion in the operation, $k_{d-m-t_d}$ increases, and the transit time is shortened.

If $k_{sd-m-ts} \leq d_{3-2}$, indicating that the early diastole of ear pulse wave rises and exceeds a normal range, then $k_{d-m-t_d}$ needs to be corrected, and the correction result is noted as $k_{d-m-t_d-1}$.

$k_{d-m-t_d-1} = k_{d-m-t_d} - (d_{3-2} - k_{sd-m-ts}) \times 75/100$; if $k_{d-m-t_d} \leq d_3$, indicated that the ear pulse wave is disturbed, then

$$k_{d-m-t_{d-toe}} = \frac{\int_{t_{s-toe}}^{t_{s-toe}+t_{d-toe}} h\,dt}{t_{d-toe} h_{max-toe}}$$

, $k_{d-m-t_d-1} = d_3$. $d_3 = 0.02$ to $0.14$, preferably is $0.08$; $d_{3-2} = 1.21$ to $1.31$, preferably is $1.26$.

$k_{d-m-t_d-toe}$ is the ratio of the average height of the d-toe max-toe toe pulse wave diastole to the maximum height $h_{max-toe}$, $t_{s-toe}$ represents the systolic time of the toe pulse wave, and $t_{d-toe}$ represents the diastolic time of the toe pulse wave. If $k_{d-m-t_d-toe} \leq d_3$, then $k_{d-m-t_d-toe} = d_3$. The properties of $k_{d-m-t_d-toe}$ and $k_{d-m-t_d}$ are same.

$k_{d-m-a} = (k_{d-m-t_d-1} + k_{d-m-t_d-toe})/2$, two variables from the ear and toe pulse waves with the same property are combined, and the average value is taken as a correction variable; if the pulse wave diastole has an irregular change, the $k_{d\text{-}m\text{-}a}$ is corrected.

If

$$|k_{sd\text{-}m\text{-}0}\text{-}k_{sd\text{-}m\text{-}ts}|\geq40 \text{ and } (k_{sd\text{-}m\text{-}0}+k_{sd\text{-}m\text{-}ts})/2\geq k_{sd\text{-}m\text{-}2} \text{ and } k_{sd\text{-}m\text{-}ts}\geq d_{3\text{-}2},$$

then

$$k_{d\text{-}m\text{-}a} = \left(k_{d\text{-}m\text{-}t_d\text{-}1} + k_{d\text{-}m\text{-}t_{d\text{-}toe}} + \left(k_{sd\text{-}m\text{-}0} + k_{sd\text{-}m\text{-}ts}\right)/2 - k_{sd\text{-}m\text{-}2}\right)/2.$$

In the state that the blood volume is normal and the body temperature of the sensor placement site is also normal, $b_3$ is not applicable. That is, if $c_4<k_{d\text{-}m\text{-}a}<c_5$, then set $b_3$=0. $c_4$=($d_4$+($age$-14)/8)/100, $d_4$=23 to 35, preferably is 29; $c_5$=($d_5$+($age$-14)/8)/100, $d_5$=27 to 39, preferably is 33.

In an extremely low or high blood pressure state, the information of diastolic period is unstable, and $b_3$ is not applicable. That is, if $k_{sd\text{-}m\text{-}0}<d_6$ or $k_{sd\text{-}m\text{-}2}>d_7$, then set $b_3$=0. $d_6$=0.97 to 1.03, preferably is 1.00; $d_7$=1.52 to 1.58, preferably is 1.55.

In a normal blood pressure state, when the blood volume decreases or the body temperature of the sensor placement site decreases, $b_3$ takes 67% of a positive value. That is, if $k_{d\text{-}m\text{-}0}\geq d_6$+0.10 and $k_{sd\text{-}m\text{-}2}\leq d_8$ and $k_{d\text{-}m\text{-}a}\leq c_4$, then $b_3$=($c_4$-$k_{d\text{-}m\text{-}a}$)×67/100. $d_8$=1.42 to 1.48, preferably is 1.45.

In relatively low or high blood pressure states, when the blood volume decreases or the body temperature of the sensor placement site decreases, $b_3$ takes 50% of a positive value. That is, if
$$\begin{cases} d_6 \leq k_{sd\text{-}m\text{-}0} < d_6 + 0.10 \\ k_{d\text{-}m\text{-}a} \leq c_4 \end{cases}$$
or
$$\begin{cases} d_8 < k_{sd\text{-}m\text{-}2} < d_7 \\ k_{d\text{-}m\text{-}a} \leq c_4 \end{cases}$$
, then $b_3$=($c_4$-$k_{d\text{-}m\text{-}a}$)×50/100;

In a normal blood pressure state, when the blood volume increases or the body temperature of the sensor placement site rises, $b_3$ takes 62% of a negative value. That is, if $k_{sd\text{-}m\text{-}0}\geq d_6$+0.10 and $k_{sd\text{-}m\text{-}2}\leq d_8$ and $k_{d\text{-}m\text{-}a}\geq c_5$, then $b_3$=($c_5$-$k_{d\text{-}m\text{-}a}$)×62/100;

In a state of relatively low or high blood pressure, when the blood volume increases or the body temperature of the sensor placement site increases, $b_3$ takes 45% of the negative value. That is, if
$$\begin{cases} d_6 \leq k_{sd\text{-}m\text{-}0} < d_6 + 0.10 \\ k_{d\text{-}m\text{-}a} \geq c_5 \end{cases}$$
or
$$\begin{cases} d_8 < k_{sd\text{-}m\text{-}2} < d_7 \\ k_{d\text{-}m\text{-}a} \geq c_5 \end{cases}$$
, then $b_3$=($c_5$-$k_{d\text{-}m\text{-}a}$)×45/100.

Fourth correction variable $b_4$:

The correction variables obtained in the step S4 further include a fourth correction variable $b_4$, which is used for correcting $T_d$ in the case that the peripheral blood vessel dilation causes the lower limb blood pressure (relative to the radial artery blood pressure) to decrease. The applicable range of $b_4$ is $b_4$>0, and if $b_4$ is larger, the lower limb blood pressure is much lowered relative to the radial artery blood pressure.

Contraction and expansion of peripheral blood vessels may cause the peak of the toe pulse wave to move back and forth on a time axis. If $t_{max\text{-}toe}\geq t_{ch\text{-}toe}$, then
$$k_{s\text{-}t\text{-}toe} = \frac{t_{max\text{-}toe} + t_{ch\text{-}toe} + 400}{\left(t_{s\text{-}toe} + 200\right)\times 2}$$
otherwise

$$k_{s-t-toe} = \frac{t_{max-toe} + 200}{t_{s-toe} + 200}$$ .

$k_{s-t-toe}$ is the ratio of the time from a start point to a peak of the toe pulse wave to the time of the systole, and 200 is an adjustment coefficient. When the highest point of the peak moves back beyond the midpoint, that is, when $t_{max-toe} \geq t_{ch-toe}$, $k_{s-r-toe}$ is corrected; when the value of $k_{s-t-toe}$ is large, indicating that the toe blood vessels dilate and the lower limb blood pressure decreases. That is, if $k_{s-t-toe} > 0.8$, then $b_4 = k_{s-t-roe} - 0.8$. If $k_{s-t-toe} \leq 0.8$, $b_4$ is not applicable, then set $b_4 = 0$.

Fifth correction variable $b_5$;

The correction variables obtained in the step S4 further include a fifth correction variable $b_5$, the role and property of $b_5$ are the same as those of the $b_4$, and $b_5$ is used for correcting $T_d$ in the case that the lower limb blood pressure

$$k_{s-m-toe} = \frac{\int_0^{t_{s-toe}} hdt}{t_{s-toe} h_{max-toe}}$$

decreases relative to the radial artery blood pressure. , $k_{s-m-toe}$ is the ratio of the average height of the toe pulse wave systole to the maximum height $h_{max-toe}$, if $k_{s-m-toe}$ is large, indicating that the toe pulse wave peak is broad and flat, suggesting that the toe blood vessels dilate, and the lower limb blood pressure decreases relative to the radial artery.

When the toe blood vessels do not dilate, $b_5$ is not applicable. That is, if $k_{s-m-toe} < d_9$, then set $b_5 = 0$. $d_9 = 0.67$ to 0.73, preferably is 0.7.

When the toe blood vessels dilate and the highest point of the pulse wave peak shifts backwards beyond the midpoint, $b_5$ takes a positive value. That is, if $k_{s-m-toe} \geq d_9$ and $k_{s-t-toe} \leq 0.8$, then $b_5 = k_{s-m-toe} - d_9$.

When the toe blood vessels dilate and the highest point of the pulse wave peak does not exceed the midpoint, $b_5$ takes half of the positive value. That is, if $k_{s-m-toe} \geq d_9$ and $k_{s-t-toe} < 0.8$, then $b_5 = (k_{s-m-toe} - d_9)/2$.

Sixth correction variable $b_6$;

The correction variables obtained in the step S4 further include a sixth correction variable $b_6$, which represents a relative change in the area of two pulse waves, and is used for correcting $T_d$ when the toe blood vessels dilate and the lower limb blood pressure decreases relative to the radial artery blood pressure. The applicable range of $b_6$ is $b_6 > 0$;

$$k_{s-m-toe-ear} = \frac{h_{max} \int_0^{t_{s-toe}} hdt + (t_s + t_{s-toe}) \times 100}{h_{max-toe} \int_0^{t_s} hdt + (t_s + t_{s-toe}) \times 100}$$

, $k_{s-m-toe-ear}$ is the ratio of the area of the toe pulse wave systole to the area of the ear pulse wave systole, and 100 is the adjustment coefficient; $k_{s-m-toe-ear}$ has the same role and property as those of $k_{ts-toe-ear}$.

When the toe wave area is smaller than the ear wave area, the toe blood vessels have no relative dilation, and $b_6$ is not applicable. That is, if $k_{s-m-toe-ear} < 1.0$, then set $b_6 = 0$.

Under the first precondition that the toe area is greatly larger than the ear area, toe blood vessels dilate more, and $c_6$ takes a constant of 1.08 as the maximum value for use. That is, if $k_{s-m-toe-ear} > 1.08$, then set $c_6 = 1.08$.

If the shape of the ear pulse wave is normal, $b_6$ takes the maximum correction value. That is, if $t_s > 220$ and $k_{sd-m-0} > 0.88$, then $b_6 = c_6 - 1.0$.

If the ear pulse wave appears as a very sharp forward-inclined triangle or the waveform is very narrow, representing that the shape of the ear pulse wave is severely irregular. At this time, the relative change between two pulse waves is amplified, the correction value needs to be reduced for use, and $b_6$ takes 1/3 of the maximum correction value. That is, if $t_s < 160$ or $k_{sd-m-0} < 0.80$, then $b_6 = (c_6 - 1.0) \times 0.34$.

When the irregularity of the shape of the ear pulse wave is not too severe, $b_6$ takes 2/3 of the maximum correction value. That is, if $160 < t_s \leq 220$ or $0.80 < k_{sd-m-0} \leq 0.88$, then $b_6 = (c_6 - 1.0) \times 0.67$.

Under the second precondition that the toe area is larger than the ear area, relative dilatation of the toe blood vessels is not too severe, and $c_6$ takes a positive variable for use. That is, if $1.0 \leq k_{s-m-toe-ear} \leq 1.08$, then $c_6 = k_{s-m-toe-ear} - 1.0$.

If the shape of the ear pulse wave is normal, $b_6$ takes a positive variable as the correction value. That is, if $t_s > 220$ and $k_{sd-m-0} > 0.88$, then $b_6 = c_6$.

If the shape of the ear pulse wave is severely irregular, the relative change between the two pulse waves is amplified, the correction value needs to be reduced for use, and $b_6$ takes 1/3 of a positive variable. That is, if $t_s<160$ or $k_{sd-m-0}\leq0.80$, then $b_6=c_6\times0.34$.

When the irregularity of the ear pulse wave is not too severe, $b_6$ takes 2/3 of a positive variable. That is, if $160<t_s\leq220$ or $0.80<k_{sd-m-0}\leq0.88$, then $b_6=c_6\times0.67$.

Seventh correction variable $b_7$;

The correction variables obtained in the step S4 further include a seventh correction variable $b_7$, the role and property of $b_7$ are the same as those of $b_6$, and $b_7$ represents the relative change of the systolic time of two pulse waves.

$$k_{ts-toe-ear} = \frac{t_{s-toe}+825}{t_s+825}$$

, $k_{ts-toe-ear}$ is the ratio of the time of systole on the toe pulse wave to the time of systole on the ear pulse wave, and 825 is the adjustment coefficient; increase in $k_{ts-toe-ear}$ suggests that the toe blood vessels dilate, and the lower limb blood pressure decreases relative to the radial artery blood pressure.

When the toe blood vessels have no relative dilation, $b_7$ is not applicable. That is, if $k_{ts-toe-ear}<1.0$, then set $b_7=0$.

Under the first precondition that toe blood vessels have severely relative dilation comparing to radial blood vessels, $c_7$ takes a constant of 1.08 as the maximum value for use. That is, if $k_{ts-toe-ear}>1.08$, then set $c_7=1.08$.

If the shape of the ear pulse wave is normal, $b_7$ takes the maximum correction value. That is, if $t_s>220$ and $k_{sd-m-0}>0.88$, then $b_7=c_7-1.0$.

If the shape of the ear pulse wave is severely irregular, the relative change between the two pulse waves is amplified, the correction value needs to be reduced for use, and $b_7$ takes 1/3 of the maximum correction value. That is, if $t_s<160$ or $k_{sd-m-0}<0.80$, then $b_7=(c_7-1.0)_\times0.34$.

If the irregularity of the shape of the ear pulse wave is not too severe, $b_7$ takes 2/3 of the maximum correction value. That is, if $160<t_s\leq220$ or $0.80<k_{sd-m-0}\leq0.88$, then $b_7=(c_7-1.0)\times0.67$.

Under the second precondition that the toe systolic time is greater than the ear systolic time, the relative dilatation of the toe blood vessels is not too severe comparing to that of radial blood vessels, and $c_7$ takes a positive variable for use. That is, if $1.0\leq k_{ts-roe-ear}\leq1.08$, then $c_7=k_{ts-toe-ear}-1.0$.

If the shape of the ear pulse wave is normal, $b_7$ takes a positive variable as the correction value. That is, if $t_s>220$ and $k_{sd-m-0}>0.88$, then $b_7=c_7$.

If the irregularity of the shape of the ear pulse wave is too severe, $b_7$ takes 1/3 of a positive variable. That is, if $t_s<160$ or $k_{sd-m-0}\leq0.80$, then $b_7=c_7\times0.34$.

If the irregularity of the shape of the ear pulse wave is not too severe, $b_7$ takes 2/3 of the positive variable. That is, if $160<t_s\leq220$ or $0.80<k_{sd-m-0}\leq0.88$, then $b_7=c_7\times0.67$.

$$B = \sum_{i=1}^{7} b_i$$

The correction matrix in the step S5 is . Where if $b_i=0$, indicating that $b_i$ is not applicable. The step S6 is specifically: continuously acquiring the correction matrices in 8 cardiac cycles, and using the average value of the 8 matrices to overcome the disturbance of respiratory fluctuation, where the 8 matrices are selected by recursion, and the oldest matrix is eliminated each time when a new matrix is calculated. A correction method is: $T_{dmb}=T_{dm}(1-B_m)$;

$$B_m = \frac{1}{8}\sum_{i=1}^{8} B_i \qquad T_{dm} = \frac{1}{8}\sum_{i=1}^{8} T_{di}$$

where , , $B_i$ is the correction matrix in the i-th cardiac cycle, and $T_{di}$ is $T_d$ in the i-th cardiac cycle.

Embodiment 2:

A method for correcting PTT associated with systolic blood pressure, includes the following steps:

S1) detecting a pulse wave at an ear in each cardiac cycle in real time and obtaining the following data: the height of an aortic valve closure point on the ear pulse wave denoted as $h_{sd}$, that is, the height at a junction between the systolic and diastolic phases on the ear pulse wave, the systolic time of the ear pulse wave denoted as $t_s$, the diastolic time of the ear pulse wave denoted as $t_d$, and the maximum height of the ear pulse wave denoted as $h_{max}$;

S2) detecting the pulse wave at a toe in each cardiac cycle in real time and obtaining the following data: the systolic time of the toe pulse wave denoted as $t_{s-toe}$, the diastolic time of the toe pulse wave denoted as $t_{d-toe}$, the maximum height of the toe pulse wave denoted as $h_{max\text{-}toe}$, the time interval between the starting point to the midpoint of the peak of the toe pulse wave denoted as $t_{ch\text{-}toe}$, and the time interval between the starting point to the highest point of the peak of the toe pulse wave denoted as $t_{max\text{-}toe}$, where the midpoint of the peak refers to the midpoint of arising edge turning point and a falling edge turning point at the peak; the definition of the midpoint of the peak can be understood by referring to the literature YAN CHEN,CHANGYUN WEN,GUOCAI TAO, and MIN BI *Continuous and Non-invasive Measurement of Systolic and Diastolic Blood Pressure by One Mathematical Model with the Same Model Parameters and Two Separate Pulse Wave Velocities.*

S3) calculating the PTT associated with systolic blood pressure denoted as $T_s$, and the definition can be understood by referring to the literature YAN CHEN, CHANGYUN WEN, GUOCAI TAO, and MIN BI *Continuous and Non-invasive Measurement of Systolic and Diastolic Blood Pressure by One Mathematical Model with the Same Model Parameters and Two Separate Pulse Wave Velocities*; $h$ is the amplitude of the ear pulse wave or the toe pulse wave in a longitudinal direction;

S4) by using the data in the same cardiac cycle acquired through step S1 and step S2, calculating a few correction variables in the cardiac cycle;

S5) according to the correction variables in the cardiac cycle acquired in step S4, calculating a correction matrix in the cardiac cycle; and

S6) continuously acquiring the correction matrices in a plurality of cardiac cycles, and correcting the $T_s$ acquired in the step S3.

By the method, the PTT associated with systolic blood pressure is determined by a time difference of an ear pulse wave and a toe pulse wave in the same cardiac cycle, and a few correction variables are extracted based on the pulse wave features, then a correction matrix is acquired to perform adaptive correction on the irregular change of pulse wave transit time. The corrected transit time can be used with available mathematical models for continuously and accurately measuring systolic blood pressure in each cardiac cycle in a clinical setting.

First correction variable $a_1$:

The correction variables obtained in the step S4 include a first correction variable $a_1$, $a_1$ is used for correcting the type II changes in $T_s$ in a hypotensive state, the applicable range of $a_1$ is $a_1>0$, and if $a_1$ is larger, the blood pressure is lower.

$$k_{sd\text{-}m\text{-}0} = \frac{t_s h_{sd}}{\int_0^{t_s} hdt};$$

$k_{sd\text{-}m\text{-}0}$ represents the ratio of $h_{sd}$ to the average height of the ear pulse wave. In some cases, under a hypotensive state, the pulse wave peak appears as a forward-inclined triangle. When $h_{sd}$ decreases a lot, $k_{sd\text{-}m\text{-}0}$ becomes smaller, indicating that the waveform at the end of the aortic systole is much lower, the continuous power for pushing wave transit is insufficient, and the transit time is prolonged. In this state, the diastolic information is unstable and should not be used. $d_1$=76 to 84, preferably is 80; $d_{1\text{-}2}$=104 to 112, preferably is 108.

When the continuous power for pushing pulse wave transit is insufficient, the transit time $T_s$ is prolonged and needed be corrected by $a_1$. That is, if $d1 \le k_{sd\text{-}m\text{-}0} \le d_{1\text{-}2}$, then $a_1=(d_{1\text{-}2}-k_{sd\text{-}m\text{-}0})\times 0.50$;

When the continuous power for pushing pulse wave transit is seriously insufficient, the transit time $T_s$ is prolonged a lot, and $a_1$ takes the upper limit value for correction. That is, if $k_{sd\text{-}m\text{-}0}<d_1$, then $a_1=28\times 0.50$;

When the continuous power for pushing pulse wave transit is sufficient, $T_s$ does not need to be corrected, and $a_1$ is not applicable. That is, if $k_{sd\text{-}m\text{-}0}>d_{1\text{-}2}$, then $a_1=0$.

Second correction variable $a_2$:

The correction variables obtained in the step S4 also include a second correction variable $a_2$, $a_2$ is used for correcting the type II changes in $T_s$ in a hypertensive state and in a change process from a normotensive state to the hypertensive state, the applicable range of $a_2$ is $a_2>0$, and if $a_2$ is larger, the systolic blood pressure is higher.

$$k_{sd\text{-}m\text{-}ts} = \frac{t_s h_{sd}}{\int_{t_s}^{2t_s} hdt}$$

$k_{sd\text{-}m\text{-}ts}$ represents the ratio of $h_{sd}$ to the average height of the $t_s$ to $2t_s$ segments of the ear pulse wave diastole, and is used for determining the irregular change of the pulse wave diastole. For example, in the thoracoabdominal surgery, the upward pulling hook causes the aortic stress to change, so that the amplitude of the ear pulse

$$k_{sd-m-2} = \frac{2t_s h_{sd}}{\int_0^{2t_s} hdt}$$

wave diastole is reduced, and the $k_{sd-m-ts}$ becomes larger. , $k_{sd-m-2}$ represents the ratio of $h_{sd}$ to the average height of the ear pulse wave 0 to $2t_s$ segments, includes the information of systolic and partial diastolic waveform, and is mainly used for a hypertensive state and a change process from a normotensive state to the hypertensive state, such as increase of heart rate and blood pressure caused by tracheal intubation. In the process of change from the normotensive state to the hypertensive state, the peak of the ear pulse wave gradually appears as an equilateral triangle or a backward-inclined triangle, $h_{sd}$ gradually increases, and $k_{sd-m-2}$ gradually becomes larger; in the hypertensive state, the entire ear pulse wave appears as an equilateral triangle or a backward-inclined triangle, $h_{sd}$ rises a lot, and $k_{sd-m-2}$ becomes very large; the peaks (i.e., the maximal blood pressure) of the triangles of the above two waveforms is very short in duration, the continuous power corresponding to the maximal blood pressure is insufficient, and the transit time $T_s$ is relatively prolonged.

If

$$|k_{sd-m-0}-k_{sd-m-ts}|\geq 40 \text{ and } (k_{sd-m-0}+k_{sd-m-ts})/2 \geq k_{sd-m-2},$$

then

$$k_{sd-m}=2\times k_{sd-m-2}-(k_{sd-m-0}+k_{sd-m-ts})/2,$$

otherwise $k_{sd-m}=k_{sd-m-2}$;

If the waveform of the ear pulse wave diastole has an irregular change, for example, if the upward pulling hook of the thoracoabdominal surgery causes the aortic stress to change, and the form of the pulse wave diastole changes significantly, the $k_{sd-m}$ is corrected, otherwise $k_{sd-m}=k_{sd-m-2}$. $d_2$=1.17 to 1.27, preferably is 1.22.

If $k_{sd-m}>(d_2+(age-14)/15/100)$, where $age$ is age, $age \geq 14$ years old, indicating that the entire ear pulse wave or the peak thereof becomes an equilateral triangle or a backward-inclined triangle, the continuous power corresponding to the maximal blood pressure is insufficient, the transit time $T_s$ is relatively prolonged, and $a_2$ is needed for correction, then $a_2=k_{sd-m}-(d_2+(age-14)|15/100)$.

If $k_{sd-m}\leq(d_2+(age-14)/15/100)$, the peak of the pulse wave is flat, the continuous power corresponding to the maximal blood pressure is sufficient, and $a_2$ is not needed for correction, then set $a_2=0$.

Third correction variable $a_3$:

The correction variables obtained in the step S4 further include a third correction variable $a_3$, which is used for correcting $T_s$ in a state that the blood volume changes or the body temperature of a sensor placement site changes.

$$k_{d-m-t_d} = \frac{\int_{t_s}^{t_s+t_d} hdt}{t_d h_{max}}$$

, $k_{d-m-t_d}$ is the ratio of the average height of the ear pulse wave diastole to the maximum height $h_{max}$. The blood volume decreases when a patient fasts and drinks less water before surgery, $k_{d-m-t_d}$ decreases, the transit time is prolonged, when the blood volume increases due to blood transfusion and IV transfusion in the operation, $k_{d-m-t_d}$ increases, and the transit time is shortened.

If $k_{sd-m-ts}\leq d_{3-2}$, indicating that the early diastole of the ear pulse wave rises and exceeds a normal range, $k_{d-m-t_d}$ needs to be corrected, and the correction result is noted as $k_{d-m-t_d-1}$.

$k_{d-m-t_d-1}= k_{d-m-t_d} -(d_{3-2}-k_{sd-m-ts})\times 75/100$ ; if $k_{d-m-td} \leq d_3$, indicated that the ear pulse wave is disturbed, then

$$k_{d-m-t_{d-toe}} = \frac{\int_{t_{s-toe}}^{t_{s-toe}+t_{d-toe}} hdt}{t_{d-toe} h_{max-toe}}$$

$k_{d-m-td-1} = d_3$. $d_3$=0.02 to 0.14, preferably is 0.08; $d_{3-2}$=1.21 to 1.31, preferably is 1.26. $k_{d-m-t_{d-toe}}$ is the ratio of the average height of the toe pulse wave diastole to the maximum height $h_{max-toe}$, $t_{s-toe}$ represents the systolic time of the toe pulse wave, and $t_{d-toe}$ represents the diastolic time of the toe pulse wave. If $k_{d-m-t_{d-toe}} \leq d_3$,

then $k_{d-m-t_d-toe} = d_3 \cdot k_{d-m-t_d-toe}$ and $k_{d-m-t_d}$ have the same role and property.

$k_{d-m-a} = (k_{d-m-t_d-1} + k_{d-m-t_d-toe})/2$, two variables from the ear and toe pulse waves with the same property are combined, and the average value is taken as the variable for correcting $T_s$. If the pulse wave diastole has an irregular change, $k_{d-m-a}$ is corrected.

If

$$|k_{sd-m-0} - k_{sd-m-ts}| \geq 40 \text{ and } (k_{sd-m-0} + k_{sd-m-ts})/2 \geq k_{sd-m-2} \text{ and } k_{sd-m-ts} \geq d_{3-2},$$

then

$$k_{d-m-a} = \left( k_{d-m-t_d-1} + k_{d-m-t_{d-toe}} + \left( k_{sd-m-0} + k_{sd-m-ts} \right)/2 - k_{sd-m-2} \right)/2.$$

In the state that the blood volume is normal and the body temperature of the sensor placement site is also normal, $a_3$ is not applicable. That is, if $c_4 < k_{d-m-a} < c_5$, then set $a_3=0$. $c_4=(d_4+(age-14)/8)/100$, $d_4=23$ to 35, preferably is 29; $c_5=(d_5+(age-14)/8)/100$, $d_5=27$ to 39, preferably is 33.

In extremely low or high blood pressure states, the information of diastolic period is unstable, and $a_3$ is not applicable. That is, if $k_{sd-m-0} < d_6$ or $k_{sd-m-2} > d_7$, then set $a_3=0$. $d_6=0.97$ to 1.03, preferably is 1.00; $d_7=1.52$ to 1.58, preferably is 1.55.

In a normal blood pressure state, when the blood volume decreases or the body temperature of the sensor placement site decreases, $a_3$ takes 67% of a positive value. That is, if $k_{sd-m-0} \geq d_6+0.10$ and $k_{sd-m-2} \leq d_8$ and $k_{d-m-a} \leq c_4$, then $a_3=(c_4-k_{d-m-a}) \times 67/1\,00$. $d_8=1.42$ to 1.48, preferably is 1.45.

In relatively low or high blood pressure states, when the blood volume decreases or the body temperature of the sensor placement site decreases, $a_3$ takes 50% of a positive value. That is, if
$$\begin{cases} d_6 \leq k_{sd-m-0} < d_6 + 0.10 \\ k_{d-m-a} \leq c_4 \end{cases}$$
or

$$\begin{cases} d_8 < k_{sd-m-2} < d_7 \\ k_{d-m-a} \leq c_4 \end{cases}$$
, then $a_3=(c_4-k_{d-m-a}) \times 50/100$;

In a normal blood pressure state, when the blood volume increases or the body temperature of the sensor placement site increases, $a_3$ takes 62% of a negative value. That is, if $k_{sd-m-0} \geq d_6+0.10$ and $k_{sd-m-2} \leq d_8$ and $k_{d-m-a} \geq c_5$, then $a_3=(c_5-k_{d-m-a}) \times 62/100$;

In a state of relatively low or high blood pressure, when the blood volume increases or the body temperature of the sensor placement site increases, $a_3$ takes 45% of the negative value. That is, if
$$\begin{cases} d_6 \leq k_{sd-m-0} < d_6 + 0.10 \\ k_{d-m-a} \geq c_5 \end{cases}$$
or

$$\begin{cases} d_8 < k_{sd-m-2} < d_7 \\ k_{d-m-a} \geq c_5 \end{cases}$$
, then $a_3=(c_5-k_{d-m-a}) \times 45/100$.

Fourth correction variable $a_4$:

The correction variables obtained in the step S4 further include a fourth correction variable $a_4$, which is used for correcting $T_s$ in the case that the peripheral blood vessel dilation causes the lower limb blood pressure (relative to the radial artery blood pressure) to decrease. The applicable range of $a_4$ is $a_4>0$, and if $a_4$ is greater, the lower limb blood pressure is much lowered relative to the radial artery blood pressure.

Contraction and expansion of peripheral blood vessels may cause the peak of the toe pulse wave to move

$$k_{s-t-toe} = \frac{t_{max-toe} + t_{ch-toe} + 400}{(t_{s-toe} + 200) \times 2}$$

back and forth on a time axis. If $t_{max-toe} \geq t_{ch-toe}$, then otherwise

$$k_{s-t-toe} = \frac{t_{\max-toe} + 200}{t_{s-toe} + 200}.$$

$k_{s-t-toe}$ is the ratio of the time from the start point to the peak of the toe pulse wave to the time of the systole, and 200 is an adjustment coefficient. When the highest point of the peak moves back beyond the midpoint, that is, when $t_{max-toe} \geq t_{ch-toe}$, $k_{s-t-toe}$ is corrected; when the value of $k_{s-t-toe}$ is relatively large, indicating that the toe blood vessels dilate and the lower limb blood pressure decreases. That is, if $k_{s-t-toe} > 0.8$, then $a_4 = k_{s-t-toe} - 0.8$ If $k_{s-t-toe} \leq 0.8$, $a_4$ is not applicable, then set $a_4 = 0$.

Fifth correction variable $a_5$;

The correction variables obtained in the step S4 further include a fifth correction variable $a_5$, the role and property of $a_5$ are the same as those of the $a_4$, and $a_5$ is used for correcting $T_s$ in the case that the lower limb blood pressure

$$k_{s-m-toe} = \frac{\int_0^{t_{s-toe}} h \, dt}{t_{s-toe} h_{\max-toe}}$$

decreases relative to the radial artery blood pressure. , $k_{s-m-toe}$ is the ratio of the average height of the toe pulse wave systole to the maximum height $h_{max-toe}$, if the $k_{s-m-toe}$ is large, indicating that the toe pulse wave peak is broad and flat, suggesting that the toe blood vessels dilate, and the lower limb blood pressure decreases relative to the radial artery. $a_5$ has the same action and property as those of $a_4$.

When the toe blood vessels do not dilate, $a_5$ is not applicable. That is, if $k_{s-m-toe} < d_9$, then set $a_5 = 0$. $d_9 = 0.67$ to 0.73, preferably is 0.7.

When the toe blood vessels dilate and the highest point of the pulse wave peak shifts backward beyond the midpoint, $a_5$ takes a positive value. That is, if $k_{s-m-toe} \geq d_9$ and $k_{s-t-toe} \leq 0.8$, then $a_5 = k_{s-m-toe} - d_9$.

When the toe blood vessels dilate and the highest point of the pulse wave peak does not exceed the midpoint, $a_5$ takes half of the positive value. That is, if $k_{s-m-toe} \geq d_9$ and $k_{s-t-toe} < 0.8$, then $a_5 = (k_{s-m-toe} - d_9)/2$.

Sixth correction variable $a_6$;

The correction variables obtained in the step S4 further include a sixth correction variable $a_6$, which represents a relative change in the area of two pulse waves, and is used for correcting $T_s$ when the toe blood vessels dilate and the lower limb blood pressure decreases relative to the radial artery blood pressure. The applicable range of $a_6$ is $a_6 > 0$.

$$k_{s-m-toe-ear} = \frac{h_{\max} \int_0^{t_{s-toe}} h \, dt + (t_s + t_{s-toe}) \times 100}{h_{\max-toe} \int_0^{t_s} h dt + (t_s + t_{s-toe}) \times 100}$$

, $k_{s-m-toe-ear}$ is the ratio of the area of the toe pulse wave systole to the area of the ear pulse wave systole, and 100 is the adjustment coefficient; $k_{s-m-toe-ear}$ has the same role and property as those of $k_{ts-toe-ear}$.

When the toe wave area is smaller than the ear wave area, the toe blood vessels have no relative dilation, and $a_6$ is not applicable. That is, if $k_{s-m-toe-ear} < 1.0$, then set $a_6 = 0$.

Under the first precondition that the toe area is larger than the ear area, toe blood vessels dilate more, and $c_6$ takes a constant of 1.08 as the maximum value for use. That is, if $k_{s-m-toe-ear} > 1.08$, then set $c_6 = 1.08$.

If the shape of the ear pulse wave is normal, $a_6$ takes the maximum correction value. That is, if $t_s > 220$ and $k_{sd-m-0} > 0.88$, then $a_6 = c_6 - 1.0$.

If the ear pulse wave appears as a very sharp forward-inclined triangle or the waveform is very narrow, indicating that the ear pulse waveform is severely irregular. At this time, the relative change between the two pulse waves is amplified, the correction value needs to be reduced for use, and $a_6$ takes 1/3 of the maximum correction value. That is, if $t_s < 160$ or $k_{sd-m-0} < 0.80$, then $a_6 = (c_6 - 1.0) \times 0.34$.

When the irregularity of the shape of the ear pulse wave is not too severe, $a_6$ takes 2/3 of the maximum correction value. That is, if $160 < t_s \leq 220$ or $0.80 < k_{sd-m-0} \leq 0.88$, then $a_6 = (c_6 - 1.0) \times 0.67$.

Under the second precondition that the toe area is larger than the ear area, the relative dilatation of the toe blood vessels is not too severe, and $c_6$ takes a positive variable for use. That is, if $1.0 \leq k_{s-m-toe-ear} \leq 1.08$, then $c_6 = k_{s-m-toe-ear} - 1.0$.

If the shape of the ear pulse wave is normal, $a_6$ takes a positive variable as the correction value. That is, if

$t_s$>220 and $k_{sd-m-0}$>0.88, then $a_6$=$c_6$.

If the shape of the ear pulse wave is severely irregular, the relative change between the two pulse waves is amplified, the correction value needs to be reduced for use, and $a_6$ takes 1/3 of the positive variable. That is, if $t_s$<160 or $k_{sd-m-0}$≤0.80, then $a_6$=$c_6$×0.34.

When the irregularity of the ear pulse wave is not too severe, $a_6$ takes 2/3 of the positive variable. That is, if 160<$t_s$≤220 or 0.80<$k_{sd-m-0}$≤0.88, then $a_6$=$c_6$×0.67.

Seventh correction variable $a_7$;

The correction variables obtained in the step S4 further include a seventh correction variable $a_7$, the role and property of $a_7$ are the same as those of $a_6$, and $a_7$ represents the relative change of the systolic time of two pulse waves.

$$k_{ts-toe-ear} = \frac{t_{s-toe} + 825}{t_s + 825}$$

, is the ratio of the time of systole on the toe pulse wave to the time of the systole on the ear pulse wave, and 825 is an adjustment coefficient; increase in $k_{ts-toe-ear}$ suggests that the toe blood vessels dilate, and the lower limb blood pressure decreases relative to the radial artery blood pressure.

When the toe blood vessels have no relative dilation, $a_7$ is not applicable. That is, if $k_{ts-toe-ear}$<1.0, then set $a_7$=0.

Under the first precondition that the toe blood vessels have severely relative dilation comparing to radial blood vessels, $c_7$ takes a constant of 1.08 as the maximum value for use. That is, if $k_{ts-toe-ear}$>1.08, then set $c_7$=1.08.

If the form of the ear pulse wave is normal, $a_7$ takes the maximum correction value. That is, if $t_s$>220 and $k_{sd-m-0}$>0.88, then $a_7$=$c_7$-1.0.

If the shape of the ear pulse wave is severely irregular, the relative change between the two pulse waves is amplified, the correction value needs to be reduced for use, and $a_7$ takes 1/3 of the maximum correction value. That is, if $t_s$<160 or $k_{sd-m-0}$≤0.80, then $a_7$=($c_7$-1.0)×0.34.

If the irregularity of the shape of the ear pulse wave is not too severe, $a_7$ takes 2/3 of the maximum correction value. That is, if 160<$t_s$≤220 or 0.80<$k_{sd-m-0}$≤0.88, then $a_7$=($c_7$-1.0)×0.67.

Under the second precondition that the toe systolic time is greater than the ear systolic time, the relative dilatation of the toe blood vessels is not too severe comparing to that of radial blood vessels, and $c_7$ takes a positive variable for use. That is, if 1.0≤$k_{ts-toe-ear}$≤1.08, then $c_7$=$k_{ts-toe-ear}$-1.0.

If the shape of the ear pulse wave is normal, $a_7$ takes a positive variable as the correction value. That is, if $t_s$>220 and $k_{sd-m-0}$>0.88, then $a_7$=$c_7$.

If the irregularity of the shape of the ear pulse wave is too severe, $a_7$ takes 1/3 of the positive variable. That is, if $t_s$<160 or $k_{sd-m-0}$≤0.80, then $a_7$=$c_7$×0.34.

If the irregularity of the shape of the ear pulse wave is not too severe, $a_7$ takes 2/3 of the positive variable. That is, if 160<$t_s$≤220 or 0.80<$k_{sd-m-0}$≤0.88, then $a_7$=$c_7$×0.67.

$$A = \sum_{i=1}^{7} a_i$$

The correction matrix in the step S5 is , where if $a_i$=0, indicating that $a_i$ is not applicable. The step S6 is specifically: continuously acquiring the correction matrices in 8 cardiac cycles, and using the average value of the 8 matrices to overcome the disturbance of respiratory fluctuation, where the 8 matrices are selected by recursion, and the oldest matrix is eliminated each time when a new matrix is calculated. The correction method is: $T_{sma}$=$T_{sm}$(1-$A_m$);

$$A_m = \frac{1}{8}\sum_{i=1}^{8} A_i \quad T_{sm} = \frac{1}{8}\sum_{i=1}^{8} T_{si}$$

where . $A_i$ is the correction matrix in the $i$-th cardiac cycle, and $T_{si}$ is $T_s$ in the $i$-th cardiac cycle.

Finally, it should be noted that the above embodiments are only used for illustrating the technical solution of the present invention, rather than limiting the present invention.

**Claims**

1. A method for obtaining a corrected value of the pulse wave transit time in the context of hypotension, hypertension, normal blood pressure changing to hypertension, blood volume changes or temperature of a sensor placement site changes, blood pressure of the lower limbs lower than that of of the upper limbs, **characterized in that**, comprising the following steps:

S1) detecting a pulse wave at an ear in each cardiac cycle in real time and obtaining the following data: the height of an aortic valve closure point on an ear pulse wave denoted as $h_{sd}$, the systolic time of the ear pulse wave denoted as $t_s$, the diastolic time of the ear pulse wave denoted as $t_d$, and the maximum height of the ear pulse wave denoted as $h_{max}$;

S2) detecting the pulse wave at a toe in each cardiac cycle in real time and obtaining the following data: the systolic time of a toe pulse wave denoted as $t_{s-toe}$, the diastolic time of the toe pulse wave denoted as $t_{d-toe}$, the maximum height of the toe pulse wave denoted as $h_{max-toe}$, the time interval between starting point to a midpoint of the systolic peak of the toe pulse wave denoted as $t_{ch-toe}$, the time interval between the starting point to the highest point of the systolic peak of the toe pulse wave denoted as $t_{max-toe}$, wherein the midpoint of the peak refers to the midpoint of arising edge turning point and a falling edge turning point at the peak;

S3) calculating the pulse wave transit time associated with diastolic blood pressure denoted as $T_d$, or calculating the pulse wave transit time associated with systolic blood pressure denoted as $T_s$, wherein $T_d$ refers to a time difference between the starting point of the ear pulse wave and the starting point of the toe pulse wave in a cardiac cycle, wherein $T_s$ refers to a time difference between the aortic valve closure point on the ear pulse wave and the aortic valve closure point on the toe pulse wave in a cardiac cycle, and $h$ is the amplitude of the ear pulse wave or the toe pulse wave in a ordinate direction;

S4) by using the data in the same cardiac cycle acquired through the step S 1 and the step S2, calculating a plurality of correction variables in the cardiac cycle;

S5) according to the correction variables $b_1 \sim b_7$ or $a_1 \sim a_7$ in the cardiac cycle acquired in the step S4, calculating a total correction value in the cardiac cycle; and

S6) continuously acquiring the total correction values in a plurality of cardiac cycles, and correcting the $T_d$ or $T_s$ acquired in the step S3,

wherein the correction variables in the step S4 include: $b_1$ and $a_1$ to correct $T_d$ or $T_s$ under hypotension ; $b_2$ to correct $T_d$ under hypertension; $a_2$ to correct $T_s$ under hypertension or normal blood pressure changing to hypertension; $b_3$ and $a_3$ to correct $T_d$ or $T_s$ when the blood volume changes or the temperature of a sensor placement site changes; $b_4$, $b_5$, $b_6$, $b_7$ and $a_4$, $a_5$, $a_6$, $a_7$ to correct $T_d$ or $T_s$ when the blood pressure of the lower limbs is lower than that of the upper limbs;

wherein when calculating $T_d$ in the step S3, the total correction value in the step S5 is $B = \sum_{i-1}^{7} b_i$ ; wherein $b_i$ is the i-th correction variable,

when calculating $T_s$ in the step S3, the total correction value in the step S3 is $A = \sum_{i=1}^{7} a_i$ , $a_i$ is the $i$-th correction variable,

wherein when calculating $T_d$ in the step S3, the first correction variable $b_1$ is calculated by the following formulas:

if $d_{1-b} \leq k_{sd-m-0} \leq d_{1-2-b}$, then $b_1 = (d_{1-2-b} - k_{sd-m-0}) \times 0.4$; if $k_{sd-m-0} < d_{1-b}$, then $b_1 = 24 \times 0.4$; if $k_{sd-m-0} > d_{1-2-b}$, then

$$k_{sd-m-0} = \frac{t_s h_{sd}}{\int_o^{t_s} h dt}$$

$b_1 = 0$; wherein $d_{1-b} = 74$ to $82$, $d_{1-2-b} = 98$ to $106$, and

when calculating $T_s$ in the step S3, the first correction variable $a_1$ is calculated by the following formulas:

if $d_1 \leq k_{sd-m-0} \leq d_{1-2}$, then $a_1 = (d_{1-2} - k_{sd-m-0}) \times 0.50$; if $k_{sd-m-0} < d_1$, then $a_1 = 28 \times 0.50$; and

$$k_{sd=m-0} = \frac{t_s h_{sd}}{\int_0^{t_s} h dt}$$

if $k_{sd-m-0} > d_{1-2}$, then $a_1 = 0$; wherein , $d_1 = 76$ to $84$, and $d_{1-2} = 104$ to $112$,

wherein when calculating $T_d$ in the step S3, the second correction variable $b_2$ is calculated by the following formulas:

if $k_{sd-m} > (d_{2-b} + (age-14)/15/100)$, then $b_2 = (k_{sd-m} - (d_{2-b} + (age-14)/15/100)) \times 0.5$;
if $k_{sd-m} \leq (d_{2-b} + (age-14)/15/100)$, then $b_2 = 0$;

wherein $d_{2\text{-}b}$=1.33 to 1.43, *age* is age, if $|k_{sd\text{-}m\text{-}0}\text{-}k_{sd\text{-}m\text{-}ts}|\geq 40$ and $(k_{sd\text{-}m\text{-}0}+k_{sd\text{-}m\text{-}ts})/2\geq k_{sd\text{-}m\text{-}2}$, then $k_{sd\text{-}m}$=$2\times k_{sd\text{-}m\text{-}2}\text{-}(k_{sd\text{-}m\text{-}0}+k_{sd\text{-}m\text{-}ts})/2$, otherwise $k_{sd\text{-}m}$=$k_{sd\text{-}m\text{-}2}$;

$$k_{sd-m-0} = \frac{t_s h_{sd}}{\int_0^{t_s} hdt}, \quad k_{sd-m-ts} = \frac{t_s h_{sd}}{\int_{t_s}^{2t_s} hdt}, \quad k_{sd-m-2} = \frac{2t_s h_{sd}}{\int_0^{2t_s} hdt},$$

when calculating $T_s$ in the step S3, the second correction variable $c2$ is calculated by the following formulas:

if $k_{sd\text{-}m}$>$(d_2+(age\text{-}14)115/100)$, then $a_2$=$k_{sd\text{-}m}\text{-}(d_2+(age\text{-}14)/15/100)$;
if $k_{sd\text{-}m}\leq(d_2+(age\text{-}14)115/100)$, then $a_2$=0;
wherein if $|k_{sd\text{-}m\text{-}0}\text{-}k_{sd\text{-}m\text{-}ts}|\geq 40$ and $(k_{sd\text{-}m\text{-}0}+k_{sd\text{-}m\text{-}ts})/2\geq k_{sd\text{-}m\text{-}2}$, then

$$k_{sd-m-0} = \frac{t_s h_{sd}}{\int_0^{t_s} hdt}$$

$k_{sd\text{-}m}$=$2\times k_{sd\text{-}m\text{-}2}\text{-}(k_{sd\text{-}m\text{-}0}+k_{sd\text{-}m\text{-}ts})/2$ otherwise $k_{sd\text{-}m}$=$k_{sd\text{-}m\text{-}2}$;

$$k_{sd-m-ts} = \frac{t_s h_{sd}}{\int_{t_s}^{2t_s} hdt}, \quad k_{sd-m-2} = \frac{2t_s h_{sd}}{\int_0^{2t_s} hdt}$$

*age* is age, and $d_2$ =1.17 to 1.27,

wherein when calculating $T_d$ in the step S3, the third correction variable $b_3$ is calculated by the following formulas:

if $c_4$<$k_{d\text{-}m\text{-}a}$<$c_5$, then $b_3$=0;
if $k_{sd\text{-}m\text{-}0}$<$d_6$ or $k_{sd\text{-}m\text{-}2}$>$d_7$, then $b_3$=0;
if $k_{sd\text{-}m\text{-}0}\geq d_6$+0.10, $k_{sd\text{-}m\text{-}2}\leq d_8$ and $k_{d\text{-}m\text{-}a}\leq c_4$, then $b_3$=$(c_4\text{-}k_{d\text{-}m\text{-}a})\times 67/100$;

if $\begin{cases} d_6 \leq k_{sd-m-0} < d_6 + 0.10 \\ \quad k_{d-m-a} \leq c_4 \end{cases}$ or $\begin{cases} d_8 < k_{sd-m-2} < d_7 \\ \quad k_{d-m-a} \leq c_4 \end{cases}$, then $b_3$=$(c_4\text{-}k_{d\text{-}m\text{-}a})\times 50/100$;

if $k_{d\text{-}m\text{-}0}\geq d_6$+0.10 and $k_{sd\text{-}m\text{-}2}\leq d_8$ and $k_{d\text{-}m\text{-}a}\geq c_5$, then $b_3$=$(c_5\text{-}k_{d\text{-}m\text{-}a})\times 62/100$;

if $\begin{cases} d_6 \leq k_{sd-m-0} < d_6 + 0.10 \\ \quad k_{d-m-a} \geq c_5 \end{cases}$ or $\begin{cases} d_8 < k_{sd-m-2} < d_7 \\ \quad k_{d-m-a} \geq c_5 \end{cases}$, then $b_3$=$(c_5\text{-}k_{d\text{-}m\text{-}a})\times 45/100$;

wherein if $|k_{sd\text{-}m\text{-}0}\text{-}k_{d\text{-}m\text{-}ts}|\geq 40$ and $(k_{sd\text{-}m\text{-}0}+k_{sd\text{-}m\text{-}ts})/2\geq k_{sd\text{-}m\text{-}2}$ and $k_{sd\text{-}m\text{-}ts}\geq d_{3\text{-}2}$, then $k_{d\text{-}m\text{-}a}$=$(k_{d\text{-}m\text{-}t_d\text{-}1}+k_{d\text{-}m\text{-}t_d\text{-}toe}+(k_{d\text{-}m\text{-}0}+k_{sd\text{-}m\text{-}ts})/2\text{-}k_{sd\text{-}m\text{-}2})/2$, otherwise $k_{d\text{-}m\text{-}a}$=$(k_{d\text{-}m\text{-}t_d\text{-}1}+k_{d\text{-}m\text{-}t_d\text{-}toe})/2$;
if $k_{sd\text{-}m\text{-}ts}\leq d_{3\text{-}2}$, then $k_{d\text{-}m\text{-}t_d\text{-}1}$ = $k_{d\text{-}m\text{-}t_d}$ -$(d_{3\text{-}2}$ - $k_{sd\text{-}m\text{-}ts})\times 75/100$ ; if $k_{d\text{-}m\text{-}t_d}\leq d_3$, then $k_{d\text{-}m\text{-}t_d\text{-}1}$ = $d_3$; if $k_{d\text{-}m\text{-}t_d\text{-}toe}\leq d_3$, then $k_{d\text{-}m\text{-}t_d\text{-}toe}$ = $d_3$;

$$k_{d-m-t_d} = \frac{\int_{t_s}^{t_s+t_d} hdt}{t_d h_{\max}}, \quad k_{sd-m-0} = \frac{t_s h_{sd}}{\int_0^{t_s} hdt}, \quad k_{sd-m-2} = \frac{2t_s h_{sd}}{\int_0^{2t_s} hdt}, \quad k_{sd-m-ts} = \frac{t_s h_{sd}}{\int_{t_s}^{2t_s} hdt},$$

$$k_{d-m-t_{d-toe}} = \frac{\int_{t_{s-toe}}^{t_{s-toe}+t_{d-toe}} h\, dt}{t_{d-toe} h_{\max-toe}}$$ ; and

$c_4$=$(d_4+(age\text{-}14)/8)/100$, $d_4$=23 to 35, $c_5$=$(d_5+(age\text{-}14)/8)/100$, $d_3$=21 to 39, $d_6$=0.97 to 1.03, $d_7$=1.52 to 1.58, $d_8$=1.42 to 1.48, $d_{3\text{-}2}$=1.21 to 1.31, $d_3$=0.02 to 0.14, and *age* is age, when calculating $T_s$ in the step S3, the third correction variable $a_3$ is calculated by the following formulas:

if $c_4 < k_{d\text{-}m\text{-}a} < c_5$, then $a_3 = 0$;

if $k_{sd\text{-}m\text{-}0} < d_6$ or $k_{sd\text{-}m\text{-}2} > d_7$, then $a_3 = 0$;

if $k_{sd\text{-}m\text{-}0} \geq d_6 + 0.10$ and $k_{sd\text{-}m\text{-}2} \leq d_8$ and $k_{d\text{-}m\text{-}a} \leq c_4$, then $a_3 = (c_4 - k_{d\text{-}m\text{-}a}) \times 67/100$;

if $\begin{cases} d_6 \leq k_{sd-m-0} < d_6 + 0.10 \\ k_{d-m-a} \leq c_4 \end{cases}$ or $\begin{cases} d_8 < k_{sd-m-2} < d_7 \\ k_{d-m-a} \leq c_4 \end{cases}$, then

$a_3 = (c_4 - k_{d\text{-}m\text{-}a}) \times 50/100$;

if $k_{sd\text{-}m\text{-}0} \geq d_6 + 0.10$ and $k_{sd\text{-}m\text{-}2} \leq d_8$ and $k_{d\text{-}m\text{-}a} \geq c_5$, then $a_3 = (c_5 - k_{d\text{-}m\text{-}a}) \times 62/100$;

if $\begin{cases} d_6 \leq k_{sd-m-0} < d_6 + 0.10 \\ k_{d-m-a} \geq c_5 \end{cases}$ or $\begin{cases} d_8 < k_{sd-m-2} < d_7 \\ k_{d-m-a} \geq c_5 \end{cases}$, then

$a_3 = (c_5 - k_{d\text{-}m\text{-}a}) \times 45/100$;

wherein if $|k_{sd\text{-}m\text{-}0} - k_{sd\text{-}m\text{-}ts}| \geq 40$ and $(k_{sd\text{-}m\text{-}0} + k_{sd\text{-}m\text{-}ts})/2 \geq k_{sd\text{-}m\text{-}2}$ and $k_{sd\text{-}m\text{-}ts} \geq d_{3\text{-}2}$, then $k_{d\text{-}m\text{-}a} = (k_{d\text{-}m\text{-}td\text{-}1} + k_{d\text{-}m\text{-}td\text{-}toe} + (k_{sd\text{-}m\text{-}0} + k_{sd\text{-}m\text{-}ts})/2 - k_{sd\text{-}m\text{-}2})/2$, otherwise $k_{d\text{-}m\text{-}a} = (k_{d\text{-}m\text{-}td\text{-}1} + k_{d\text{-}m\text{-}td\text{-}toe})/2$;

if $k_{sd\text{-}m\text{-}ts} \leq d_{3\text{-}2}$, then $k_{d\text{-}m\text{-}td\text{-}1} = k_{d\text{-}m\text{-}td} - (d_{3\text{-}2} - k_{sd\text{-}m\text{-}ts}) \times 75/100$; if $k_{d\text{-}m\text{-}td} \leq d_3$, then $k_{d\text{-}m\text{-}td\text{-}1} = d_3$; if $k_{d\text{-}m\text{-}td\text{-}toe} \leq d_3$, then $k_{d\text{-}m\text{-}td\text{-}toe} = d_3$;

$$k_{d-m-t_d} = \frac{\int_{t_s}^{t_s+t_d} h\,dt}{t_d h_{\max}} \quad , \quad k_{sd-m-0} = \frac{t_s h_{sd}}{\int_0^{t_s} h\,dt} \quad , \quad k_{sd-m-2} = \frac{2t_s h_{sd}}{\int_0^{2t_s} h\,dt} \quad , \quad k_{sd-m-ts} = \frac{t_s h_{sd}}{\int_{t_s}^{2t_s} h\,dt}$$

,

$$k_{d-m-t_{d-toe}} = \frac{\int_{t_{s-toe}}^{t_{s-toe}+t_{d-toe}} h\,dt}{t_{d-toe} h_{\max-toe}}$$ ; and

$c_4 = (d_4 + (age-14)/8)/100$, $d_4 = 23$ to $35$, $c_5 = (d_5 + (age-14)/8)/100$, $d_5 = 27$ to $39$, $d_6 = 0.97$ to $1.03$, $d_7 = 1.52$ to $1.58$, $d_8 = 1.42$ to $1.48$, $d_{3\text{-}2} = 1.21$ to $1.31$, $d_3 = 0.02$ to $0.14$, and $age$ is age,

wherein when calculating $T_d$ in the step S3, the fourth correction variable $b_4$ is calculated by the following formulas:

if $k_{s\text{-}t\text{-}rte} > 0.8$, then $b_4 = k_{s\text{-}t\text{-}toe} - 0.8$;

if $k_{s\text{-}t\text{-}toe} \leq 0.8$, then $b_4 = 0$;

$$k_{s-t-toe} = \frac{t_{\max-toe} + t_{ch-toe} + 400}{(t_{s-toe} + 200) \times 2}$$

wherein if $t_{max\text{-}toe} \geq t_{ch\text{-}toe}$, then , otherwise

$$k_{s-t-toe} = \frac{t_{\max-toe} + 200}{t_{s-toe} + 200}$$

,

when calculating $T_s$ in the step S3, the fourth correction variable $a_4$ is calculated by the following formulas: if $k_{s\text{-}t\text{-}rte} > 0.8$, then $a_4 = k_{s\text{-}t\text{-}toe} - 0.8$;

if $k_{s\text{-}t\text{-}toe} \leq 0.8$, then $a_4 = 0$;

$$k_{s-t-toe} = \frac{t_{\max-toe} + t_{ch-toe} + 400}{(t_{s-toe} + 200) \times 2}$$

wherein if $t_{max\text{-}toe} \geq t_{ch\text{-}toe}$, then , otherwise

$$k_{s-t-toe} = \frac{t_{max-toe} + 200}{t_{s-toe} + 200}$$

wherein when calculating $T_d$ in the step S3, the fifth correction variable $b_5$ is calculated by the following formulas:

if $k_{s-m-toe} < d_9$, then $b_5 = 0$;
if $k_{s-m-toe} \geq d_9$ and $k_{s-t-rte} > 0.8$, then $b_5 = k_{s-m-toe} - d_9$;
if $k_{s-m-toe} \geq d_9$ and $k_{s-t-toe} < 0.8$, then $b_5 = (k_{s-m-toe} - d_9)/2$;

$$k_{s-m-toe} = \frac{\int_0^{t_{s-toe}} h\,dt}{t_{s-toe}h_{max-toe}}$$

wherein $d_9 = 0.67$ to $0.73$, and

when calculating $T_s$ in the step S3, the fifth correction variable $a_5$ is calculated by the following formulas:

if $k_{s-m-toe} < d_9$, then $a_5 = 0$;
if $k_{s-m-toe} \geq d_9$ and $k_{s-t-toe} \geq 0.8$, then $a_5 = k_{s-m-toe} - d_9$;
if $k_{s-m-toe} \geq d_9$ and $k_{s-t-toe} < 0.8$, then $a_5 = (k_{s-m-toe} - d_9)/2$;

$$k_{s-m-toe} = \frac{\int_0^{t_{s-toe}} h\,dt}{t_{s-toe}h_{max-toe}}$$

wherein $d_9 = 0.67$ to $0.73$,

when calculating $T_d$ in the step S3, the sixth correction variable $b_6$ is calculated by the following formulas:

if $k_{s-m-toe-ear} < 1.0$, then $b_6 = 0$;
when $k_{s-m-toe-ear} > 1.08$, then $c_6 = 1.08$, meantime, if $t_s > 220$ and $k_{sd-m-0} > 0.88$, then $b_6 = c_6 - 1.0$, if $t_s < 160$ or $k_{sd-m-0} < 0.80$, then $b_6 = (c_6 - 1.0) \times 0.34$, if $160 < t_s \leq 220$ or $0.80 < k_{sd-m-0} \leq 0.88$, then $b_6 = (c_6 - 1.0) \times 0.67$;
when $1.0 \leq k_{s-m-toe-ear} \leq 1.08$, then $c_6 = k_{s-m-toe-ear} - 1.0$, meantime, if $t_s > 220$ and $k_{sd-m-0} > 0.88$, then $b_6 = c_6$, if $t_s < 160$ or $k_{sd-m-0} < 0.80$, then $b_6 = c_6 \times 0.34$, if $160 < t_s \leq 220$ or $0.80 < k_{sd-m-0} \leq 0.88$, then $b_6 = c_6 \times 0.67$;

$$k_{s-m-toe-ear} = \frac{h_{max} \int_0^{t_{s-toe}} h\,dt + (t_s + t_{s-toe}) \times 100}{h_{max-toe} \int_0^{t_s} h\,dt + (t_s + t_{s-toe}) \times 100}$$

wherein

$$k_{sd-m-0} = \frac{t_s h_{sd}}{\int_0^{t_s} h\,dt}$$

and

when calculating $T_s$ in the step S3, the sixth correction variable $a_6$ is calculated by the following formulas:

if $k_{s-m-toe-ear} < 1.0$, then $a_6 = 0$;
when $k_{s-m-toe-ear} > 1.08$, then $c_6 = 1.08$, meantime, if $t_s > 220$ and $k_{sd-m-0} > 0.88$, then $a_6 = c_6 - 1.0$, if $t_s < 160$ or $k_{sd-m-0} < 0.80$, then $a_6 = (c_6 - 1.0) \times 0.34$, if $160 < t_s \leq 220$ or $0.80 < k_{sd-m-0} \leq 0.88$, then $a_6 = (c_6 - 1.0) \times 0.67$;
when $1.0 \leq k_{s-m-toe-ear} \leq 1.08$, then $c_6 = k_{s-m-toe-ear} - 1.0$, meantime, if $t_s > 220$ and $k_{sd-m-0} > 0.88$, then $a_6 = c_6$, if $t_s < 160$ or $k_{sd-m-0} \leq 0.80$, then $a_6 = c_6 \times 0.34$, if $160 < t_s \leq 220$ or $0.80 < k_{sd-m-0} \leq 0.88$, then $a_6 = c_6 \times 0.67$;

wherein

$$k_{s-m-toe-ear} = \frac{h_{\max}\int_0^{t_{s-toe}} h\,dt + \left(t_s + t_{s-toe}\right)\times 100}{h_{\max-toe}\int_0^{t_s} h\,dt + \left(t_s + t_{s-toe}\right)\times 100}, \quad k_{sd-m-0} = \frac{t_s h_{sd}}{\int_0^{t_s} h\,dt},$$

when calculating $T_d$ in the step S3, the seventh correction variable $b_7$ is calculated by the following formulas:

if $k_{ts-toe-ear}<1.0$, then $b_7=0$;
when $k_{ts-toe-ear}>1.08$, then $c_7=1.08$, meantime, if $t_s>220$ and $k_{sd-m-0}>0.88$, then $b_7=c_7-1.0$, if $t_s<160$ or $k_{sd-m-0}<0.80$, then $b_7=(c_7-1.0)\times 0.34$, if $160<t_s\leq 220$ or $0.80<k_{sd-m-0}\leq 0.88$, then $b_7=(c_7-1.0)\times 0.67$;
when $1.0\leq k_{ts-toe-ear}\leq 1.08$, then $c_7=k_{ts-toe-ear}-1.0$, meantime, if $t_s>220$ and $k_{sd-m-0}>0.88$, then $b_7=c_7$, if $t_s<160$ or $k_{sd-m-0}\leq 0.80$, then $b_7=c_7\times 0.34$, if $160<t_s\leq 220$ or $0.80<k_{sd-m-0}\leq 0.88$, then $b_7=c_7\times 0.67$;

$$k_{ts-toe-ear} = \frac{t_{s-toe}+825}{t_s+825}$$

wherein , and

$$k_{sd-m-0} = \frac{t_s h_{sd}}{\int_0^{t_s} h\,dt},$$

wherein when calculating $T_s$ in the step S3, the seventh correction variable $a_7$ is calculated by the following formulas:

if $k_{ts-toe-ear}<1.0$, then $a_7=0$;
when $k_{ts-toe-ear}>1.08$, then $c_7=1.08$, meantime, if $t_s>220$ and $k_{sd-m-0}>0.88$, then $a_7=c_7-1.0$, if $t_s<160$ or $k_{sd-m-0}<0.80$, then $a_7=(c_7-1.0)\times 0.34$, if $160<t_s\leq 220$ or $0.80<k_{sd-m-0}\leq 0.88$, then $a_7=(c_7-1.0)\times 0.67$;
when $1.0\leq k_{ts-toe-ear}<1.08$, then $c_7=k_{ts-toe-ear}-1.0$, meantime, if $t_s>220$ and $k_{sd-m-0}>0.88$, then $a_7=c_7$, if $t_s<160$ or $k_{sd-m-0}<0.80$, then $a_7=c_7\times 0.34$, if $160<t_s\leq 220$ or $0.80<k_{sd-m-0}\leq 0.88$, then $a_7=c_7\times 0.67$;
wherein

$$k_{ts-toe-ear} = \frac{t_{s-toe}+825}{t_s+825}, \quad k_{sd-m-0} = \frac{t_s h_{sd}}{\int_0^{t_s} h\,dt}.$$

2. The method according to claim 1, **characterized in that**, in the step S6, the total correction values in 8 cardiac cycles are continuously acquired; when calculating $T_d$ in the step S3, the corrected value of pulse wave transit time associated with diastolic blood pressure $T_{dmb}$ is calculated by: $T_{dmh}=T_{dm}(1-B_m)$; wherein

$$B_m = \frac{1}{8}\sum_{i=1}^{8} B_i, \quad T_{dm} = \frac{1}{8}\sum_{i=1}^{8} T_{di}$$

, $B_i$ is the total correction value in the i-th cardiac cycle, and $T_{di}$ is $T_d$ in the i-th cardiac cycle,
when calculating $T_s$ in the step S3, the corrected value of pulse wave transit time associated with systolic blood

$$A_m = \frac{1}{8}\sum_{i=1}^{8} A_i \ , \quad T_{sm} = \frac{1}{8}\sum_{i=1}^{8} T_{si}$$

pressure $T_{sma}$ is calculated by: $T_{sma}=T_{sm}(1-A_m)$; wherein , $A_i$ is the total correction value in the i-th cardiac cycle, and $T_{si}$ is $T_s$ in the i-th cardiac cycle.

## Patentansprüche

**1.** - Verfahren zum Erlangen eines korrigierten Werts der Pulswellen-Transitzeit im Zusammenhang mit Hypotonie, Hypertonie, normalem Blutdruck, der sich zu Hypertonie ändert, Blutvolumenänderungen oder Temperaturänderungen einer Sensorplatzierungsstelle, Blutdruck der unteren Gliedmaßen niedriger als der der oberen Gliedmaßen, **dadurch gekennzeichnet, dass** es die folgenden Schritte umfasst:

S1) Erkennen einer Pulswelle an einem Ohr in jedem Herzzyklus in Echtzeit und Erlangen der folgenden Daten: die Höhe eines Aortenklappen-Schließpunkts an einer Ohrpulswelle, bezeichnet als $h_{sd}$, die systolische Zeit der Ohrpulswelle, bezeichnet als $t_s$, die diastolische Zeit der Ohrpulswelle, bezeichnet als $t_d$, und die maximale Höhe der Ohrpulswelle, bezeichnet als $h_{max}$;

S2) Erkennen der Pulswelle an einer Zehe in jedem Herzzyklus in Echtzeit und Erlangen der folgenden Daten: die systolische Zeit einer Zehenpulswelle, bezeichnet als $t_{s\text{-}toe}$, die diastolische Zeit der Zehenpulswelle, bezeichnet als $t_{d\text{-}toe}$, die maximale Höhe der Zehenpulswelle, bezeichnet als $h_{max\text{-}toe}$ bezeichnet, das Zeitintervall zwischen dem Startpunkt und einem Mittelpunkt der systolischen Spitze der Zehenpulswelle, bezeichnet als $t_{ch\text{-}toe}$ bezeichnet, das Zeitintervall zwischen dem Startpunkt und dem höchsten Punkt der systolischen Spitze der Zehenpulswelle, bezeichnet als $t_{max\text{-}toe}$, wobei sich der Mittelpunkt der Spitze auf den Mittelpunkt des Wendepunkts der aufsteigenden Flanke und des Wendepunkts der abfallenden Flanke an der Spitze bezieht;

S3) Berechnen der mit dem diastolischen Blutdruck assoziierten Pulswellentransitzeit, bezeichnet als $T_d$, oder Berechnen der mit dem systolischen Blutdruck verbundenen Pulswellentransitzeit, bezeichnet als $T_s$ wobei sich $T_d$ auf eine Zeitdifferenz zwischen dem Startpunkt der Ohrpulswelle und dem Startpunkt der Zehenpulswelle in einem Herzzyklus bezieht, wobei sich $T_s$ auf eine Zeitdifferenz zwischen dem Aortenklappen-Schließpunkt an der Ohrpulswelle und dem Aortenklappen-Schließpunkt an der Zehenpulswelle in einem Herzzyklus bezieht, und h die Amplitude der Ohrpulswelle oder der Zehenpulswelle in einer Ordinatenrichtung ist;

S4) durch Verwenden der Daten in demselben Herzzyklus, die durch den Schritt S1 und den Schritt S2 erfasst wurden, Berechnen einer Vielzahl von Korrekturvariablen in dem Herzzyklus;

S5) gemäß den Korrekturvariablen $b_1 \sim b_7$ oder $a_1 \sim a_7$ in dem in Schritt S4 erfassten Herzzyklus, Berechnen eines Gesamtkorrekturwerts in dem Herzzyklus; und

S6) kontinuierliches Erfassen der Gesamtkorrekturwerte in einer Vielzahl von Herzzyklen und Korrigieren des $T_d$ oder $T_s$, die in Schritt S3 erfasst werden,

wobei die Korrekturvariablen in dem Schritt S4 Folgendes beinhalten: $b_1$ und $a_1$ zum Korrigieren von $T_d$ oder $T_s$ bei Hypotonie; $b_2$ zum Korrigieren von $T_d$ bei Hypertonie; $a_2$ zum Korrigieren von $T_s$ bei Hypertonie oder normalem Blutdruck, der sich zu Hypertonie ändert; $b_3$ und $a_3$ zum Korrigieren von $T_d$ oder $T_s$, wenn sich das Blutvolumen ändert oder die Temperatur einer Sensorplatzierungsstelle ändert; $b_4$, $b_5$, $b_6$, $b_7$ und $a_4$, $a_5$, $a_6$, $a_7$ zum Korrigieren von $T_d$ oder $T_s$, wenn der Blutdruck der unteren Gliedmaßen niedriger ist als der der oberen Gliedmaßen;

$$B = \sum_{i=1}^{7} b_i$$

wobei beim Berechnen von $T_d$ in dem Schritt S3 der Gesamtkorrekturwert in dem Schritt S5 ist; wobei $b_i$ die *i-te* Korrekturvariable ist,

$$A = \sum_{i=1}^{7} a_i$$

beim Berechnen von $T_s$ in dem Schritt S3 der Gesamtkorrekturwert in dem Schritt S3 ist, $a_i$ die *i-te* Korrekturvariable ist, wobei beim Berechnen von $T_d$ in dem Schritt S3 die erste Korrekturvariable $b_1$ gemäß den folgenden Formeln berechnet wird:

wenn $d_{1-b} \leq k_{sd-m-0} \leq d_{1-2-i}$, dann $b_1 = (d_{1-2-b}-k_{sd-m-0}) \times 0,4$ ; wenn $k_{sd-m-0}<d_{1-b}$, dann $b_1=24\times0,4$; wenn

$$k_{sd-m-0} = \frac{t_s h_{sd}}{\int_o^{t_s} h dt}$$

$k_{sd-m-0}>d_{1-2-b}$, dann $b_1=0$; wobei $d_{1-b}$=74 bis 82, $d_{1-2-b}$=98 bis 106, und ,
beim Berechnen von $T_s$ in dem Schritt S3 die erste Korrekturvariable $a_1$ gemäß den folgenden Formeln berechnet wird:

wenn $d_1 \leq k_{sd-m-0} \leq d_{1-2}$, dann $a_1=(d_{1-2}-k_{sd-m-0})\times0,50$; wenn $k_{sd-m-0}<d_1$, dann $a_1=28\times0,50$; und

$$k_{sd=m-0} = \frac{t_s h_{sd}}{\int_0^{t_s} h dt}$$

wenn $k_{sd-m-0}>d_{1-2}$, dann $a_1=0$ ; wobei , $d_1$=76 bis 84, und $d_{1-2}$ =104 bis 112,
wobei beim Berechnen von $T_d$ in dem Schritt S3 die zweite Korrekturvariable $b_2$ gemäß den folgenden Formeln berechnet wird:

wenn $k_{sd-m}>(d_{2-b}+(Alter-14)/15/100)$, dann $b_2=(k_{sd-m}-(d_{2-b}+(Alter-14)/15/100))\times0,5$;
wenn $k_{sd-m}\leq(d_{2-b}+(Alter-14)/15/100)$, dann $b_2=0$;
wobei $d_{2-b}$=1,33 bis 1,43, *Alter Alter* ist, wenn $|k_{sd-m-0}-k_{sd-m-ts}|\geq40$ und $(k_{sd-m-0}+k_{sd-m-ts})/2\geq k_{sd-m-2}$,

$$k_{sd-m-0} = \frac{t_s h_{sd}}{\int_0^{t_s} h dt}$$

dann $k_{sd-m}=2\times k_{sd-m-2}-(k_{sd-m-0}+k_{sd-m-ts})/2$, sonst $k_{sd-m}=k_{sd-m-2}$; ,

$$k_{sd-m-ts} = \frac{t_s h_{sd}}{\int_{t_s}^{2t_s} h dt} \quad , \quad k_{sd-m-2} = \frac{2t_s h_{sd}}{\int_0^{2t_s} h dt}$$

,
beim Berechnen von $T_s$ in dem Schritt S3 die zweite Korrekturvariable $a_2$ gemäß den folgenden Formeln berechnet wird:

wenn $k_{sd-m}>(d_2+(Alter-14)/15/100)$, dann $a_2=k_{sd-m}-(d_2+(Alter-14)/15/100)$ ;
wenn $k_{sd-m}\leq(d_2+(Alter-14)/15/100)$, dann $a_2=0$;
wobei wenn $|k_{sd-m-0}-k_{sd-m-ts}|\geq40$ und $(k_{sd-m-0}+k_{sd-m-ts})/2\geq k_{sd-m-2}$, dann $k_{sd-m}=2\times k_{sd-m-2}-(k_{sd-m-0}+k_{sd-m-ts})/2$, sonst $k_{sd-m}=k_{sd-m-2}$;

$$k_{sd-m-0} = \frac{t_s h_{sd}}{\int_0^{t_s} h dt} \quad , \quad k_{sd-m-ts} = \frac{t_s h_{sd}}{\int_{t_s}^{2t_s} h dt} \quad , \quad k_{sd-m-2} = \frac{2t_s h_{sd}}{\int_0^{2t_s} h dt}$$

, *Alter Alter* ist, und
$d_2$ =1,17 bis 1,27,
wobei beim Berechnen von $T_d$ in dem Schritt S3 die dritte Korrekturvariable $b_3$ gemäß den folgenden Formeln berechnet wird:

wenn $c_4<k_{d-m-a}<c_5$, dann $b_3=0$;
wenn $k_{sd-m-0}<d_6$ oder $k_{sd-m-2}>d_7$, dann $b_3=0$;
wenn $k_{sd-m-0}\geq d_6+0,10$, $k_{sd-m-2}\leq d_8$ und $k_{d-m-a}\leq c_4$, dann $b_3=(c_4-k_{d-m-a})\times67/100$;

wenn $\begin{cases} d_6 \leq k_{sd-m-0} < d_6 + 0,10 \\ k_{d-m-a} \leq c_4 \end{cases}$ oder $\begin{cases} d_8 < k_{sd-m-2} < d_7 \\ k_{d-m-a} \leq c_4 \end{cases}$ , dann
$b_3=(c_4-k_{d-m-a})\times50/100$;
wenn $k_{sd-m-0}\geq d_6+0,10$ und $k_{sd-m-2}\leq d_8$ und $k_{d-m-a}\geq c_5$, dann $b_3=(c_5-k_{d-m-a})\times62/100$;

$$\begin{cases} d_6 \leq k_{sd-m-0} < d_6 + 0,10 \\ k_{d-m-a} \geq c_5 \end{cases}$$

wenn $\qquad$ oder $\begin{cases} d_8 < k_{sd-m-2} < d_7 \\ k_{d-m-a} \geq c_5 \end{cases}$ , dann $b_3 = (c_5 - k_{d-m-a}) \times 45/100$;

wobei wenn $|k_{sd-m-0} - k_{sd-m-ts}| \geq 40$ und $(k_{sd-m-0} + k_{sd-m-ts})/2 \geq k_{sd-m-2}$ und $k_{sd-m-ts} \geq d_{3-2}$, dann $k_{d-m-a} = (k_{d-m-t_d-1} + k_{d-m-t_d-Zehe} + (k_{sd-m-0} + k_{sd-m-ts})/2 - k_{sd-m-2})/2$, sonst $k_{d-m-a} = (k_{d-m-t_d-1} + k_{d-m-t_d-Zehe})/2$;

wenn $k_{sd-m-ts} \leq d_{3-2}$, dann $k_{d-m-t_d-1} = k_{d-m-t_d} - (d_{3-2} - k_{sd-m-ts}) \times 75/100$; wenn $k_{d-m-t_d} \leq d_3$, dann $k_{d-m-t_d-1} = d_3$; wenn $k_{d-m-t_d-toe} \leq d_3$, dann $k_{d-m-t_d-toe} = d_3$;

$$k_{d-m-t_d} = \frac{\int_{t_s}^{t_s+t_d} h\,dt}{t_d h_{\max}} \,, \quad k_{sd-m-0} = \frac{t_s h_{sd}}{\int_0^{t_s} h\,dt} \,, \quad k_{sd-m-2} = \frac{2t_s h_{sd}}{\int_0^{2t_s} h\,dt} \,, \quad k_{sd-m-ts} = \frac{t_s h_{sd}}{\int_{t_s}^{2t_s} h\,dt} \,,$$

$$k_{d-m-t_{d-toe}} = \frac{\int_{t_{s-toe}}^{t_{s-toe}+t_{d-toe}} h\,dt}{t_{d-toe} h_{\max-toe}} \; ; \text{ und}$$

$c_4 = (d_4 + (Alter-14)/8)/100$, $d_4 = 23$ bis 35, $c_5 = (d_5 + (Alter-14)/8)/100$, $d_5 = 27$ bis 39, $d_6 = 0,97$ bis 1,03, $d_7 = 1,52$ bis 1,58, $d_8 = 1,42$ bis 1,48, $d_{3-2} = 1,21$ bis 1,31, $d_3 = 0,02$ bis 0,14, und *Alter Alter* ist,

beim Berechnen von $T_s$ in dem Schritt S3 die dritte Korrekturvariable $a_3$ gemäß den folgenden Formeln berechnet wird:

wenn $c_4 < k_{d-m-a} < c_5$, dann $a_3 = 0$;
wenn $k_{sd-m-0} < d_6$ oder $k_{sd-m-2} > d_7$, dann $a_3 = 0$;
wenn $k_{sd-m-0} \geq d_6 + 0,10$ und $k_{sd-m-2} \leq d_8$ und $k_{d-m-a} \leq c_4$, dann $a_3 = (c_4 - k_{d-m-a}) \times 67/100$;

$$\begin{cases} d_6 \leq k_{sd-m-0} < d_6 + 0,10 \\ k_{d-m-a} \leq c_4 \end{cases}$$

wenn $\qquad$ oder $\begin{cases} d_8 < k_{sd-m-2} < d_7 \\ k_{d-m-a} \leq c_4 \end{cases}$ , dann

$a_3 = (c_4 - k_{d-m-a}) \times 50/100$;

wenn $k_{sd-m-0} \geq d_6 + 0,10$ und $k_{sd-m-2} \leq d_8$ und $k_{d-m-a} \geq c_5$, dann $a_3 = (c_5 - k_{d-m-a}) \times 62/100$;

$$\begin{cases} d_6 \leq k_{sd-m-0} < d_6 + 0,10 \\ k_{d-m-a} \geq c_5 \end{cases}$$

wenn $\qquad$ oder $\begin{cases} d_8 < k_{sd-m-2} < d_7 \\ k_{d-m-a} \geq c_5 \end{cases}$ , dann

$a_3 = (c_5 - k_{d-m-a}) \times 45/100$;

wobei wenn $|k_{sd-m-0} - k_{sd-m-ts}| \geq 40$ und $(k_{sd-m-0} + k_{sd-m-ts})/2 \geq k_{sd-m-2}$ und $k_{sd-m-ts} \geq d_{3-2}$, dann $k_{d-m-a} = (k_{d-m-t_d-1} + k_{d-m-t_d-toe} + (k_{sd-m-0} + k_{sd-m-ts})/2 - k_{sd-m-2})/2$, sonst $k_{d-m-a} = (k_{d-m-t_d-1} + k_{d-m-t_d-toe})/2$;

wenn $k_{sd-m-ts} \leq d_{3-2}$, dann $k_{d-m-t_d-1} = k_{d-m-t_d} - (d_{3-2} - k_{sd-m-ts}) \times 75/100$; wenn $k_{d-m-t_d} \leq d_3$,

dann $k_{d-m-t_d-1} = d_3$; wenn $k_{d-m-t_d-toe} \leq d_3$, dann $k_{d-m-t_d-toe} = d_3$;

$$k_{d-m-t_d} = \frac{\int_{t_s}^{t_s+t_d} h\,dt}{t_d h_{\max}} \,, \quad k_{sd-m-0} = \frac{t_s h_{sd}}{\int_0^{t_s} h\,dt} \,, \quad k_{sd-m-2} = \frac{2t_s h_{sd}}{\int_0^{2t_s} h\,dt} \,, \quad k_{sd-m-ts} = \frac{t_s h_{sd}}{\int_{t_s}^{2t_s} h\,dt} \,,$$

$$k_{d-m-t_{d-toe}} = \frac{\int_{t_{s-toe}}^{t_{s-toe}+t_{d-toe}} h\,dt}{t_{d-toe} h_{\max-toe}} \; ; \text{ und}$$

$c_4 = (d_4 + (Alter-14)/8)/100$, $d_4 = 23$ bis 35, $c_5 = (d_5 + (Alter-14)/8)/100$, $d_5 = 27$ bis 39, $d_6 = 0,97$ bis 1,03, $d_7 = 1,52$ bis 1,58, $d_8 = 1,42$ bis 1,48, $d_{3-2} = 1,21$ bis 1,31, $d_3 = 0,02$ bis

0,14, und *Alter Alter* ist,

wobei beim Berechnen von $T_d$ in dem Schritt S3 die vierte Korrekturvariable $b_4$ gemäß den folgenden Formeln berechnet wird:

wenn $k_{s\text{-}t\text{-}toe}>0,8$, dann $b_4= k_{s\text{-}t\text{-}toe}-0,8$;

wenn $k_{s\text{-}t\text{-}toe}\leq0,8$, dann $b_4=0$;

wobei wenn $t_{max\text{-}toe}\geq t_{ch\text{-}toe}$, dann $k_{s-t-toe} = \dfrac{t_{\max-toe} + t_{ch-toe} + 400}{(t_{s-toe} + 200) \times 2}$ , sonst

$$k_{s-t-toe} = \dfrac{t_{\max-toe} + 200}{t_{s-toe} + 200},$$

beim Berechnen von $T_s$ in dem Schritt S3 die vierte Korrekturvariable $a_4$ gemäß den folgenden Formeln berechnet wird: wenn $k_{s\text{-}t\text{-}toe}>0,8$, dann $a_4= k_{s\text{-}t\text{-}toe}-0,8$; wenn $k_{s\text{-}t\text{-}toe}\leq0,8$, dann $a_4=0$;

wobei wenn $t_{max\text{-}toe}\geq t_{ch\text{-}toe}$, dann $k_{s-t-toe} = \dfrac{t_{\max-toe} + t_{ch-toe} + 400}{(t_{s-toe} + 200) \times 2}$ , sonst

$$k_{s-t-toe} = \dfrac{t_{\max-toe} + 200}{t_{s-toe} + 200}.$$

wobei beim Berechnen von $T_d$ in dem Schritt S3 die fünfte Korrekturvariable $b_5$ gemäß den folgenden Formeln berechnet wird:

wenn $k_{s\text{-}m\text{-}toe}<d_9$, dann $b_5=0$;

wenn $k_{s\text{-}m\text{-}toe}\geq d_9$ und $k_{s\text{-}t\text{-}toe}\geq0,8$, dann $b_5=k_{s\text{-}m\text{-}toe}-d_9$;

wenn $k_{s\text{-}m\text{-}toe}\geq d_9$ und $k_{s\text{-}t\text{-}toe}<0, 8$, dann $b_5=(k_{s\text{-}m\text{-}toe}-d_9)/2$;

wobei $d_9=0,67$ bis $0,73$, und $k_{s-m-toe} = \dfrac{\int_0^{t_{s-toe}} h\,dt}{t_{s-toe}h_{\max-toe}}$ ,

beim Berechnen von $T_s$ in dem Schritt S3 die fünfte Korrekturvariable $a_5$ gemäß den folgenden Formeln berechnet wird:

wenn $k_{s\text{-}m\text{-}toe}<d_9$, dann $a_5=0$;

wenn $k_{s\text{-}m\text{-}toe}\geq d_9$ und $k_{s\text{-}t\text{-}toe}\geq0,8$, dann $a_5=k_{s\text{-}m\text{-}toe}-d_9$;

wenn $k_{s\text{-}m\text{-}toe}\geq d_9$ und $k_{s\text{-}t\text{-}toe}<0, 8$, dann $a_5=(k_{s\text{-}m\text{-}toe}-d_9)/2$;

wobei $d_9=0,67$ bis $0, 73$, $k_{s-m-toe} = \dfrac{\int_0^{t_{s-toe}} h\,dt}{t_{s-toe}h_{\max-toe}}$ ,

beim Berechnen von $T_d$ in dem Schritt S3 die siebte Korrekturvariable $b_6$ gemäß den folgenden Formeln berechnet wird:

wenn $k_{s\text{-}m\text{-}toe\text{-}ear}<1,0$, dann $b_6=0$;

wenn $k_{s\text{-}m\text{-}toe\text{-}ear}>1,08$, dann $c_6=1,08$, währenddessen, wenn $t_s>220$ und $k_{sd\text{-}m\text{-}0}>0,88$, dann $b_6=c_6-1,0$, wenn $t_s<160$ oder $k_{sd\text{-}m\text{-}0}<0,80$, dann $b_6=(c_6-1,0)\times0,34$, wenn $160<t_s\leq220$ oder $0,80<k_{sd\text{-}m\text{-}0}\leq0,88$, dann $b_6=(c_6-1,0)\times0,67$;

wenn $1, 0\leq k_{s\text{-}m\text{-}toe\text{-}ear}\leq1,08$, dann $c_6=k_{s\text{-}m\text{-}toe\text{-}ear}-1,0$, währenddessen, wenn $t_s>220$ und $k_{sd\text{-}m\text{-}0}>0,88$, dann $b_6=c_6$, wenn $t_s\leq160$ oder $k_{sd\text{-}m\text{-}0}\leq0,80$, dann $b_6=c_6\times0,34$, wenn $160<t_s\leq220$ oder $0, 80<k_{sd\text{-}m\text{-}0}\leq0,88$, dann $b_6=c_6\times0,67$;

$$k_{s-m-toe-ear} = \frac{h_{\max} \int_0^{t_{s-toe}} h\, dt + (t_s + t_{s-toe}) \times 100}{h_{\max-toe} \int_0^{t_s} hdt + (t_s + t_{s-toe}) \times 100}$$

wobei , und

$$k_{sd-m-0} = \frac{t_s h_{sd}}{\int_0^{t_s} hdt},$$

beim Berechnen von $T_s$ in dem Schritt S3 die sechste Korrekturvariable $a_6$ gemäß den folgenden Formeln berechnet wird:

wenn $k_{s-m-toe-ear}<1{,}0$, dann $a_6=0$;
wenn $k_{s-m-toe-ear}>1{,}08$, dann $c_6=1{,}08$, währenddessen, wenn $t_s>220$ und $k_{sd-m-0}>0{,}88$, dann $a_6=c_6-1{,}0$, wenn $t_s<160$ oder $k_{sd-m-0}<0{,}80$, dann $a_6=(c_6-1{,}0)\times 0{,}34$, wenn $160<t_s\leq 220$ oder $0{,}80<k_{sd-m-0}\leq 0{,}88$, dann $a_6=(c_6-1{,}0)\times 0{,}67$;

wenn $1{,}0\leq k_{s-m-toe-ear}\leq 1{,}08$, dann $c_6=k_{s-m-toe-ear}-1{,}0$, währenddessen, wenn $t_s>220$ und $k_{sd-m-0}>0{,}88$, dann $a_6=c_6$, wenn $t_s\leq 160$ oder $k_{sd-m-0}<0{,}80$ dann $a_6=c_6\times 0{,}34$, wenn $160<t_s\leq 220$ oder $0{,}80<k_{sd-m-0}\leq 0{,}88$, dann $a_6=c_6\times 0{,}67$;

wobei

$$k_{s-m-toe-ear} = \frac{h_{\max} \int_0^{t_{s-toe}} h\, dt + (t_s + t_{s-toe}) \times 100}{h_{\max-toe} \int_0^{t_s} hdt + (t_s + t_{s-toe}) \times 100}, \quad k_{sd-m-0} = \frac{t_s h_{sd}}{\int_0^{t_s} hdt},$$

beim Berechnen von $T_d$ in dem Schritt S3 die siebte Korrekturvariable $b_7$ gemäß den folgenden Formeln berechnet wird:

wenn $k_{ts-toe-ear}<1{,}0$, dann $b_7=0$;
wenn $k_{ts-toe-ear}>1{,}08$, dann $c_7=1{,}08$, währenddessen, wenn $t_s>220$ und $k_{sd-m-0}>0{,}88$, dann $b_7=c_7-1{,}0$, wenn $t_s<160$ oder $k_{sd-m-0}<0{,}80$, dann $b_7=(c_7-1{,}0)\times 0{,}34$, wenn $160<t_s\leq 220$ oder $0{,}80<k_{sd-m-0}\leq 0{,}88$, dann $b_7=(c_7-1{,}0)\times 0{,}67$;

wenn $1{,}0<k_{ts-toe-ear}<1{,}08$, dann $c_7=k_{ts-toe-ear}-1{,}0$, währenddessen, wenn $t_s>220$ und $k_{sd-m-0}>0{,}88$, dann $b_7=c_7$, wenn $t_s\leq 160$ oder $k_{sd-m-0}\leq 0{,}80$, dann $b_7=c_7\times 0{,}34$, wenn $160<t_s\leq 220$ oder $0{,}80<k_{sd-m-0}\leq 0{,}88$, dann $b_7=c_7\times 0{,}67$;

$$k_{ts-toe-ear} = \frac{t_{s-toe}+825}{t_s+825}$$

wobei , und

$$k_{sd-m-0} = \frac{t_s h_{sd}}{\int_0^{t_s} hdt},$$

wobei beim Berechnen von $T_s$ in dem Schritt S3 die siebte Korrekturvariable $a_7$ gemäß den folgenden Formeln berechnet wird:

wenn $k_{ts-toe-ear}<1{,}0$, dann $a_7=0$;
wenn $k_{ts-toe-ear}>1{,}08$, dann $c_7=1{,}08$, währenddessen, wenn $t_s>220$ und $k_{sd-m-0}>0{,}88$, dann $a_7=c_7-1{,}0$, wenn $t_s<160$ oder $k_{sd-m-0}<0{,}80$, dann $a_7=(c_7-1{,}0)\times 0{,}34$, wenn $160<t_s\leq 220$ oder $0{,}80<k_{sd-m-0}\leq 0{,}88$, dann $a_7=(c_7-1{,}0)\times 0{,}67$;

wenn $1{,}0\leq k_{ts-toe-ear}<1{,}08$, dann $c_7=k_{ts-toe-ear}-1{,}0$, währenddessen, wenn $t_s>220$ und $k_{sd-m-0}>0{,}88$, dann $a_7=c_7$, wenn $t_s\leq 160$ oder $k_{sd-m-0}\leq 0{,}80$, dann $a_7=c_7\times 0{,}34$, wenn $160<t_s\leq 220$ oder $0{,}80<k_{sd-m-0}\leq 0{,}88$, dann $a_7=c_7\times 0{,}67$;

wobei

$$k_{ts-toe-ear} = \frac{t_{s-toe} + 825}{t_s + 825} \;, \quad k_{sd-m-0} = \frac{t_s h_{sd}}{\int_0^{t_s} h \, dt}.$$

**2.** - Verfahren nach Anspruch 1, **dadurch gekennzeichnet, dass** in dem Schritt S6 die Gesamtkorrekturwerte in 8 Herzzyklen kontinuierlich erfasst werden; beim Berechnen von $T_d$ in dem Schritt S3 der korrigierte Wert der Pulswellentransitzeit, die mit dem diastolischen Blutdruck $T_{dmb}$ assoziiert ist, berechnet wird durch: $T_{dmb}=T_{dm}(1-B_m)$;

$$B_m = \frac{1}{8}\sum_{i=1}^{8} B_i \;, \qquad T_{dm} = \frac{1}{8}\sum_{i=1}^{8} T_{di}$$

wobei , $B_i$ der Gesamtkorrekturwert in dem *i-ten* Herzzyklus ist, und $T_{di}$ $T_d$ in dem *i-ten* Herzzyklus ist,
beim Berechnen von $T_s$ in dem Schritt S3 der korrigierte Wert der Pulswellentransitzeit, die mit dem systolischen Blutdruck $T_{sma}$ assoziiert ist, durch Folgendes berechnet wird: $T_{sma}=T_{sm}(1-A_m)$; wobei

$$A_m = \frac{1}{8}\sum_{i=1}^{8} A_i \;, \qquad T_{sm} = \frac{1}{8}\sum_{i=1}^{8} T_{si}$$

, $A_i$ der Gesamtkorrekturwert in dem *i-ten* Herzzyklus ist, und $T_{si}$ $T_s$ in dem *i-ten* Herzzyklus ist.

## Revendications

**1.** - Procédé d'obtention d'une valeur corrigée de la durée de propagation d'ondes pulsatiles dans le contexte d'hypotension, d'hypertension, de changement de pression artérielle normale en une hypertension, de changements de volume sanguin ou de changements de température d'un site de positionnement de capteur, de pression artérielle des membres inférieurs inférieure à celle des membres supérieurs, **caractérisé par le fait qu'**il comprend les étapes suivantes :

S1) détecter une onde pulsatile à une oreille dans chaque cycle cardiaque en temps réel et obtenir les données suivantes : la hauteur d'un point de fermeture de valve aortique sur une onde pulsatile d'oreille, désignée par $h_{sd}$, la durée systolique de l'onde pulsatile d'oreille, désignée par $t_s$, la durée diastolique de l'onde pulsatile d'oreille, désignée par $t_d$, et la hauteur maximale de l'onde pulsatile d'oreille, désignée par $h_{max}$ ;
S2) détecter l'onde pulsatile à un orteil dans chaque cycle cardiaque en temps réel et obtenir les données suivantes : la durée systolique d'une onde pulsatile d'orteil, désignée par $t_{s-toe}$, la durée diastolique de l'onde pulsatile d'orteil, désignée par $t_{d-toe}$, la hauteur maximale de l'onde pulsatile d'orteil, désignée par $h_{max-toe}$, l'intervalle de temps entre le point de départ et un point médian du pic systolique de l'onde pulsatile d'orteil, désigné par $t_{ch-toe}$, l'intervalle de temps entre le point de départ et le point le plus élevé du pic systolique de l'onde pulsatile d'orteil, désigné par $t_{max-toe}$, le point médian du pic se référant au point médian d'un point d'inflexion de front ascendant et d'un point d'inflexion de front descendant au pic ;
S3) calculer la durée de propagation d'onde pulsatile associée à une pression artérielle diastolique, désignée par $T_d$, ou calculer la durée de propagation d'onde pulsatile associée à une pression artérielle systolique, désignée par $T_s$, $T_d$ se référant à une différence de temps entre le point de départ de l'onde pulsatile d'oreille et le point de départ de l'onde pulsatile d'orteil dans un cycle cardiaque, $T_s$ se référant à une différence de temps entre le point de fermeture de valve aortique sur l'onde pulsatile d'oreille et le point de fermeture de valve aortique sur l'onde pulsatile d'orteil dans un cycle cardiaque, et h étant l'amplitude de l'onde pulsatile d'oreille ou de l'onde pulsatile d'orteil dans une direction d'ordonnées ;
S4) en utilisant les données du même cycle cardiaque acquises au cours de l'étape S1 et de l'étape S2, calculer une pluralité de variables de correction dans le cycle cardiaque ;
S5) en fonction des variables de correction $b_1 \sim b_7$ ou $a_1 \sim a_7$ dans le cycle cardiaque acquises à l'étape S4, calculer une valeur de correction totale dans le cycle cardiaque ; et
S6) acquérir en continu les valeurs de correction totales dans une pluralité de cycles cardiaques, et corriger $T_d$ ou $T_s$ acquis à l'étape S3,
les variables de correction à l'étape S4 comprenant : $b_1$ et $a_1$ pour corriger $T_d$ ou $T_s$ en cas d'hypotension ; $b_2$ pour corriger $T_d$ en cas d'hypertension ; $a_2$ pour corriger $T_s$ en cas d'hypertension ou de changement de pression artérielle normale en une hypertension ; $b_3$ et $a_3$ pour corriger $T_d$ ou $T_s$ en cas de changements de volume sanguin ou de changements de température d'un site de positionnement de capteur ; $b_4$, $b_5$, $b_6$, $b_7$ et $a_4$, $a_5$, $a_6$, $a_7$ pour corriger $T_d$ ou $T_s$ lorsque la pression artérielle des membres inférieurs est inférieure à celle des membres supérieurs ;

lors du calcul de $T_d$ à l'étape S3, la valeur de correction totale à l'étape S5 étant $B = \sum_{i-1}^{7} b_i$ ; $b_i$ étant la $i^{\text{ème}}$ variable de correction,

lors du calcul de $T_s$ à l'étape S3, la valeur de correction totale à l'étape S3 étant $A = \sum_{i-1}^{7} a_i$, $a_i$ étant la $i^{\text{ème}}$ variable de correction,

lors du calcul de $T_d$ à l'étape S3, la première variable de correction $b_1$ étant calculée par les formules suivantes :

si $d_{1-b} \leq k_{sd-m}-0 \leq d_{1-2-b}$, alors $b_1 = (d_{1-2-b} - k_{sd-m-0}) \times 0,4$ ; si $k_{sd-m-0} < d_{1-b}$, alors $b_1 = 24 \times 0,4$ ; si $k_{sd-m-0} > d_{1-2-b}$,

$$k_{sd-m-0} = \frac{t_s h_{sd}}{\int_o^{t_s} h dt}$$

alors $b_1 = 0$ ; $d_{1-b} = 74$ à 82, $d_{1-2-b} = 98$ à 106, et

lors du calcul de $T_s$ à l'étape S3, la première variable de correction $a_1$ étant calculée par les formules suivantes :

si $d1 \leq k_{sd-m-0} \leq d1-2$, alors $a_1 = (_{d1-2-ksd-m-0}) \times 0,50$ ; si $k_{sd-m-0} < d_1$, alors $a1 = 28 \times 0,50$ ; et

$$k_{sd=m-0} = \frac{t_s h_{sd}}{\int_0^{t_s} h dt}$$

si $k_{sd-m-0} > d_{1-2}$, alors $a_1 = 0$ ; , $d_1 = 76$ à 84, et $d_{1-2} = 104$ à 112,

lors du calcul de $T_d$ à l'étape S3, la deuxième variable de correction $b_2$ étant calculée par les formules suivantes :

si $k_{sd-m} > (d_{2-b} + (age-14)/15/100)$, alors $b_2 = (k_{sd-m} - (d_{2-b} + (age-14)/15/100)) \times 0,5$ ;
si $k_{sd-m} \leq (d_{2-b} + (age-14)/15/100)$, alors $b_2 = 0$ ;
$d_{2-b} = 1,33$ à 1,43, $age$ étant l'âge, si $|k_{sd-m-0} - k_{sd-m-ts}| \geq 40$ et $(k_{sd-m-0} + k_{sd-m-ts})/2 \geq k_{sd-m-2}$, alors $k_{sd-m} = 2 \times k_{sd-m-2} - (k_{sd-m-0} + k_{sd-m-ts})/2$, sinon $k_{sd-m} = k_{sd-m-2}$ ;

$$k_{sd-m-0} = \frac{t_s h_{sd}}{\int_0^{t_s} h dt} , \quad k_{sd-m-ts} = \frac{t_s h_{sd}}{\int_{t_s}^{2t_s} h dt} , \quad k_{sd-m-2} = \frac{2t_s h_{sd}}{\int_0^{2t_s} h dt} ,$$

lors du calcul de $T_s$ à l'étape S3, la deuxième variable de correction $a_2$ étant calculée par les formules suivantes :

si $k_{sd-m} > (d_2 + (age-14)/15/100)$, alors $a_2 = k_{sd-m} - (d_2 + (age-14)/15/100)$ ;
si $k_{sd-m} \leq (d_2 + (age-14)/15/100)$, alors $a_2 = 0$ ;
si $|k_{sd-m-0} - k_{sd-m-ts}| \geq 40$ et $(k_{sd-m-0} + k_{sd-m-ts})/2 \geq k_{sd-m-2}$, alors $k_{sd-m} = 2 \times k_{sd-m-2} - (k_{sd-m-0} + k_{sd-m-ts})/2$,

$$k_{sd-m-0} = \frac{t_s h_{sd}}{\int_0^{t_s} h dt} , \quad k_{sd-m-ts} = \frac{t_s h_{sd}}{\int_{t_s}^{2t_s} h dt} , \quad k_{sd-m-2} = \frac{2t_s h_{sd}}{\int_0^{2t_s} h dt} ,$$

sinon $k_{sd-m} = k_{sd-m-2}$ ;
age étant l'âge, et $d_2 = 1,17$ à 1,27,

lors du calcul de $T_d$ à l'étape S3, la troisième variable de correction $b_3$ est calculée par les formules suivantes :

si $C_4 < k_{d-m-a} < C_5$, alors $b_3 = 0$ ;
si $k_{sd-m}-0 < d_6$ ou $k_{sd-m}-2 > d_7$, alors $b_3 = 0$ ;
si $k_{sd-m}-0 \geq d_6 + 0,10$, $k_{sd-m}-2 \leq d_8$ et $k_{d-m}-a \leq c_4$, alors $b_3 = (c_4 - k_{d-m-a}) \times 67/100$ ;

$$\text{si} \begin{cases} d_6 \leq k_{sd-m-0} < d_6 + 0.10 \\ k_{d-m-a} \leq c_4 \end{cases} \text{ou} \begin{cases} d_8 < k_{sd-m-2} < d_7 \\ k_{d-m-a} \leq c_4 \end{cases}, \text{ alors } b_3 = (c_4 - k_{d-m-a}) \times 50/100;$$

si $k_{sd-m-0} \geq d_6 + 0{,}10$ et $k_{sd-m-2} \leq d_8$ et $k_{d-m-a} \geq c_5$, alors $b_3 = (c_5 - k_{d-m-a}) \times 62/100$;

$$\text{si} \begin{cases} d_6 \leq k_{sd-m-0} < d_6 + 0.10 \\ k_{d-m-a} \geq c_5 \end{cases} \text{ou} \begin{cases} d_8 < k_{sd-m-2} < d_7 \\ k_{d-m-a} \geq c_5 \end{cases}, \text{ alors }$$

$b_3 = (c_5 - k_{d-m-a}) \times 45/100$ ;

si $| k_{sd-m-0} - k_{sd-m-ts} | \geq 40$ et $(k_{sd-m-0} + k_{sd-m-ts})/2 \geq k_{sd-m-2}$ et $k_{sd-m-ts} \geq d3\text{-}2$, alors $k_{d-m-a} = (k_{d-m-t_d-1} + k_{d-m-t_{d-toe}} + (k_{sd-m-0} + k_{sd-m-ts})/2 - k_{sd-m-2})/2$, sinon $k_{d-m-a} = (k_{d-m-t_d-1} + k_{d-m-t_{d-toe}})/2$;

si $k_{sd-m-ts} \leq d_{3\text{-}2}$, alors $k_{d-m-t_d-1} = k_{d-m-t_d} - (d_{3\text{-}2} - k_{sd-m-ts}) \times 75/100$ ; si $k_{d-m-t_d} \leq d_3$, alors $k_{d-m-t_d-1} = d_3$ ;

$$k_{d-m-t_d} = \frac{\int_{t_s}^{t_s+t_d} hdt}{t_d h_{max}}, \qquad k_{sd-m-0} = \frac{t_s h_{sd}}{\int_0^{t_s} hdt}, \qquad k_{sd-m-2} = \frac{2t_s h_{sd}}{\int_0^{2t_s} hdt}$$

si $k_{d-m-t_{d-toe}} \leq d_3$, alors $k_{d-m-t_{d-toe}} = d_3$ ;

$$k_{sd-m-ts} = \frac{t_s h_{sd}}{\int_{t_s}^{2t_s} hdt}, \qquad k_{d-m-t_{d-toe}} = \frac{\int_{t_{s-toe}}^{t_{s-toe}+t_{d-toe}} h\, dt}{t_{d-toe} h_{max-toe}}$$

; et

$c_4 = (d_4 + (age\text{-}14)/8)/100$, $d_4 = 23$ à $35$, $c_5 = (d_5 + (age\text{-}14)/8)/100$, $d_5 = 27$ à $39$, $d_6 = 0{,}97$ à $1{,}03$, $d_7 = 1{,}52$ à $1{,}58$, $d_8 = 1{,}42$ à $1{,}48$, $d_{3\text{-}2} = 1{,}21$ à $1{,}31$, $d_3 = 0{,}02$ à $0{,}14$, et age étant l'âge, lors du calcul de $T_s$ à l'étape S3, la troisième variable de correction $a_3$ étant calculée par les formules suivantes :

si $c_4 < k_{d-m-a} < c_5$, alors $a_3 = 0$ ;

si $k_{sd-m-0} < d_6$ ou $k_{sd-m-2} > d_7$, alors $a_3 = 0$ ;

si $k_{sd-m-0} \geq d_6 + 0{,}10$ et $k_{sd-m-2} \leq d_8$ et $k_{d-m-a} \leq c_4$, alors $a_3 = (c_4 - k_{d-m-a}) \times 67/100$ ;

$$\text{si} \begin{cases} d_6 \leq k_{sd-m-0} < d_6 + 0.10 \\ k_{d-m-a} \leq c_4 \end{cases} \text{ou} \begin{cases} d_8 < k_{sd-m-2} < d_7 \\ k_{d-m-a} \leq c_4 \end{cases}, \text{ alors }$$

$a_3 = (c_4 - k_{d-m-a}) \times 50/100$;

si $k_{sd-m-0} \geq d_6 + 0{,}10$ et $k_{sd-m-2} \leq d_8$ et $k_{d-m-a} \geq c_5$, alors $a_3 = (c_5 - k_{d-m-a}) \times 62/100$ ;

$$\text{si} \begin{cases} d_6 \leq k_{sd-m-0} < d_6 + 0.10 \\ k_{d-m-a} \geq c_5 \end{cases} \text{ou} \begin{cases} d_8 < k_{sd-m-2} < d_7 \\ k_{d-m-a} \geq c_5 \end{cases}, \text{ alors } a_3 =$$

$(c_5 - k_{d-m-a}) \times 45/100$ ;

si $| k_{sd-m-0} - k_{sd-m-ts} | \geq 40$ et $(k_{sd-m-0} + k_{sd-m-ts})/2 \geq k_{sd-m-2}$ et $k_{sd-m-ts} \geq d3\text{-}2$, alors $k_{d-m-a} = (k_{d-m-t_d-1} + k_{d-m-t_{d-toe}} + (k_{sd-m-0} + k_{sd-m-ts})/2 - k_{sd-m-2})/2$, sinon $k_{d-m-a} = (k_{d-m-t_d-1} + k_{d-m-t_{d-toe}})/2$;

si $k_{sd-m-ts} \leq d_{3\text{-}2}$, alors $k_{d-m-t_d-1} = k_{d-m-t_d} - (d_{3\text{-}2} - k_{sd-m-ts}) \times 75/100$ ; si $k_{d-m-t_d} \leq d_3$, alors $k_{d-m-t_d-1} = d_3$ ; si $k_{d-m-t_{d-toe}} \leq d_3$, alors $k_{d-m-t_{d-toe}} = d_3$ ;

$$k_{d-m-t_d} = \frac{\int_{t_s}^{t_s+t_d} hdt}{t_d h_{max}} \ , \qquad k_{sd-m-0} = \frac{t_s h_{sd}}{\int_0^{t_s} hdt} \ , \qquad k_{sd-m-2} = \frac{2t_s h_{sd}}{\int_0^{2t_s} hdt} \ , \qquad k_{sd-m-ts} = \frac{t_s h_{sd}}{\int_{t_s}^{2t_s} hdt}$$

,

$$k_{d-m-t_{d-toe}} = \frac{\int_{t_{s-toe}}^{t_{s-toe}+t_{d-toe}} h\,dt}{t_{d-toe} h_{max-toe}}$$

; et

$c_4=(d_4+(age-14)/8)/100$, $d_4=23$ à $35$, $c_5=(d_5+(age-14)/8)/100$, $d_5=27$ à $39$, $d_6=0{,}97$ à $1{,}03$, $d_7=1{,}52$ à $1{,}58$, $d_8=1{,}42$ à $1{,}48$, $d_3$-$2=1{,}21$ à $1{,}31$, $d_3=0{,}02$ à $0{,}14$, et age étant l'âge,

lors du calcul de $T_d$ à l'étape S3, la quatrième variable de correction $b_4$ étant calculée par les formules suivantes :

si $k_{s-t-toe}>0{,}8$, alors $b_4=k_{s-t-toe}$-$0{,}8$ ;
si $k_{s-t-toe}\le 08$, alors $b_4=0$ ;

si $t_{max-toe}\ge t_{ch-toe}$, alors $\quad k_{s-t-toe} = \dfrac{t_{max-toe}+t_{ch-toe}+400}{\left(t_{s-toe}+200\right)\times 2}$ , sinon

$$k_{s-t-toe} = \frac{t_{max-toe}+200}{t_{s-toe}+200} \ ,$$

lors du calcul de $T_s$ à l'étape S3, la quatrième variable de correction $a_4$ étant calculée par les formules suivantes : si $k_{s-t-toe}>0{,}8$, alors $a_4=k_{s-t-toe}$-$0{,}8$ ;
si $k_{s-t-toe}\le 0{,}8$, alors $a_4=0$ ;

si $t_{max-toe}\ge t_{ch-toe}$, alors $\quad k_{s-t-toe} = \dfrac{t_{max-toe}+t_{ch-toe}+400}{\left(t_{s-toe}+200\right)\times 2}$ , sinon

$$k_{s-t-toe} = \frac{t_{max-toe}+200}{t_{s-toe}+200} \ ,$$

lors du calcul de $T_d$ à l'étape S3, la cinquième variable de correction $b_5$ étant calculée par les formules suivantes :

si $k_{s-m-toe}<d_9$, alors $b_5=0$ ;
si $k_{s-m-toe}\ge d_9$ et $k_{s-t-toe}\ge 0{,}8$, alors $b_5=k_{s-m-toe}$-$d_9$ ;
si $k_{s-m-toe}\ge d_9$ et $k_{s-t-toe}<0{,}8$, alors $b_5=(k_{s-m-toe}$-$d_9)/2$ ;

$$k_{s-m-toe} = \frac{\int_0^{t_{s-toe}} h\,dt}{t_{s-toe} h_{max-toe}} \ ,$$

où $d_9=0{,}67$ à $0{,}73$, et

lors du calcul de $T_s$ à l'étape S3, la cinquième variable de correction $a_5$ étant calculée par les formules suivantes :

si $k_{s-m-toe}<d_9$, alors $a_5=0$ ;
si $k_{s-m-toe}\ge d_9$ et $k_{s-t-toe}\ge 0{,}8$, alors $a_5=k_{s-m-toe}$-$d_9$ ;
si $k_{s-m-toe}\ge d_9$ et $k_{s-t-toe}<0{,}8$, alors $a_5=(k_{s-m-toe}$-$d_9)/2$ ;

$$k_{s-m-toe} = \frac{\int_0^{t_{s-toe}} h\,dt}{t_{s-toe} h_{max-toe}} \ ,$$

où $d_9=0{,}67$ à $0{,}73$,

lors du calcul de $Td$ à l'étape S3, la sixième variable de correction $b_6$ étant calculée par les formules suivantes :

si $k_{s\text{-}m\text{-}toe\text{-}ear}$<1,0, alors $b_6$=0 ;

lorsque $k_{s\text{-}m\text{-}toe\text{-}ear}$>1,08, alors $c_6$=1,08, entre-temps, si $t_s$>220 et $k_{sd\text{-}m\text{-}0}$>0,88, alors $b_6=c_6$-1,0, si $t_s$<160 ou $k_{sd\text{-}m\text{-}0}$<0,80, alors $b_6=(c_6$-1,0)×0,34, si 160<$t_s$≤220 ou 0, 80<$k_{sd\text{-}m\text{-}0}$≤0,88, alors $b_6=(c_6$-1,0)×0,67 ;

lorsque 1, 0≤$k_{s\text{-}m\text{-}toe\text{-}ear}$≤1,08, alors $c_6=k_{s\text{-}m\text{-}toe\text{-}ear}$-1,0, entre-temps, si $t_s$>220 et $k_{sd\text{-}m\text{-}0}$>0,88, alors $b_6=c_6$, si $t_s$≤160 ou $K_{sd\text{-}m\text{-}0}$≤0,80, alors $b_6=c_6$×0,34, si 160<$t_s$≤220 ou 0, 80<$k_{sd\text{-}m\text{-}0}$≤0,88, alors $b_6=c_6$×0,67 ;

$$k_{s-m-toe-ear} = \frac{h_{\max} \int_0^{t_{s-toe}} h\,dt + \left(t_s + t_{s-toe}\right) \times 100}{h_{\max-toe} \int_0^{t_s} h\,dt + \left(t_s + t_{s-toe}\right) \times 100}$$

où , et

$$k_{sd-m-0} = \frac{t_s h_{sd}}{\int_0^{t_s} h\,dt}$$

lors du calcul de $T_s$ à l'étape S3, la sixième variable de correction $a_6$ étant calculée par les formules suivantes :

si $k_{s\text{-}m\text{-}toe\text{-}ear}$<1,0, alors $a_6$=0 ;

lorsque $k_{s\text{-}m\text{-}toe\text{-}ear}$>1,08, alors $c_6$=1,08, entre-temps, si $t_s$>220 et $k_{sd\text{-}m\text{-}0}$>0,88, alors $a_6=c_6$-1,0, si $t_s$<160 ou $k_{sd\text{-}m\text{-}0}$<0,80, alors $a_6=(c_6$-1,0)×0,34, si 168<$t_s$≤220 ou 0, 80<$k_{sd\text{-}m\text{-}0}$≤0,88, alors $a_6=(c_6$-1,0)×0,67 ;

lorsque 1, 0≤$k_{s\text{-}m\text{-}toe\text{-}ear}$≤1, 08, alors $c_6=k_{s\text{-}m\text{-}toe\text{-}ear}$-1,0, entre-temps, si $t_s$>220 et $k_{sd\text{-}m\text{-}0}$>0,88, alors $a_6=c_6$, si $t_s$≤160 ou $k_{sd\text{-}m\text{-}0}$-0,80, alors $a_6=c_6$×0,34, si 168<$t_s$≤220 ou 0, 80<$k_{sd\text{-}m\text{-}0}$≤0,88, alors $a_6=c_6$×0,67 ;

où

$$k_{s-m-toe-ear} = \frac{h_{\max} \int_0^{t_{s-toe}} h\,dt + \left(t_s + t_{s-toe}\right) \times 100}{h_{\max-toe} \int_0^{t_s} h\,dt + \left(t_s + t_{s-toe}\right) \times 100}, \quad k_{sd-m-0} = \frac{t_s h_{sd}}{\int_0^{t_s} h\,dt},$$

lors du calcul de $T_d$ à l'étape S3, la septième variable de correction $b_7$ étant calculée par les formules suivantes :

si $k_{ts\text{-}toe\text{-}ear}$<1,0, alors $b_7$=0 ;

lorsque $k_{ts\text{-}toe\text{-}ear}$>1,08, alors $c_7$=1,08, entre-temps, si $t_s$>220 et $k_{sd\text{-}m\text{-}0}$>0,88, alors $b_7=c_7$-1,0, si $t_s$<160 ou $k_{sd\text{-}m\text{-}0}$<0,80, alors $b_7=(c_7$-1,0)×0,34, si 168<$t_s$≤220 ou 0, 80<$k_{sd\text{-}m\text{-}0\text{-}}$≤0,88, alors $b_7=(c_7$-1,0)×0,67 ;

lorsque 1,0≤$k_{ts\text{-}toe\text{-}ear}$≤1 08, alors $c_7=k_{ts\text{-}toe\text{-}ear}$-1,0, entre-temps, si $t_s$>220 et $k_{sd\text{-}m\text{-}0}$>0,88, alors $b_7=c_7$, si $t_s$≤160 ou $k_{sd\text{-}m\text{-}0}$≤0,80, alors $b_7=c_7$×0,34, si 160<$t_s$≤220 ou 0, 80<$k_{sd\text{-}m\text{-}0}$≤0,88, alors $b_7=c_7$×0,67 ;

$$k_{ts-toe-ear} = \frac{t_{s-toe} + 825}{t_s + 825} \qquad k_{sd-m-0} = \frac{t_s h_{sd}}{\int_0^{t_s} h\,dt}$$

où , et ,

lors du calcul de $T_s$ à l'étape S3, la septième variable de correction $a_7$ étant calculée par les formules suivantes :

si $k_{ts\text{-}toe\text{-}ear}$<1,0, alors $a_7$=0 ;

lorsque $k_{ts\text{-}toe\text{-}ear}$>1,08, alors $c_7$=1,08, entre-temps, si $t_s$>220 et $k_{sd\text{-}m\text{-}0}$>0,88, alors $a_7=c_7$-1,0, si $t_s$<160 ou $k_{sd\text{-}m\text{-}0}$<0,80, alors $a_7=(c_7$-1,0)×0,34, si 160<$t_s$≤220 ou 0,80<$k_{sd\text{-}m\text{-}0}$≤0,88, alors $a_7=(c_7$-1,0)×0, 67 ;

lorsque 1,0≤$k_{ts\text{-}toe\text{-}ear}$≤1,08, alors $c_7=k_{ts\text{-}toe\text{-}ear}$-1,0, entre-temps, si $t_s$>220 et $k_{sd\text{-}m\text{-}0}$>0,88, alors $a_7=c_7$, si $t_s$≤160 ou $k_{sd\text{-}m\text{-}0}$≤0,80, alors $a_7=c_7$×0,34, si 160<$t_s$≤220 ou 0,80<$k_{sd\text{-}m\text{-}0}$≤0,88, alors $a_7=c_7$×0,67 ;

$$k_{ts-toe-ear} = \frac{t_{s-toe} + 825}{t_s + 825} \; , \quad k_{sd-m-0} = \frac{t_s h_{sd}}{\int_0^{t_s} h\,dt}$$

où

.

2. - Procédé selon la revendication 1, **caractérisé par le fait que**, à l'étape S6, les valeurs de correction totales dans 8 cycles cardiaques sont acquises en continu ; lors du calcul de $T_d$ à l'étape S3, la valeur corrigée de la durée de propagation d'onde pulsatile associée à une pression artérielle diastolique $T_{dmb}$ est calculée par : $T_{dmb} = T_{dm}(1-B_m)$ ;

$$B_m = \frac{1}{8}\sum_{i=1}^{8} B_i \; , \quad T_{dm} = \frac{1}{8}\sum_{i=1}^{8} T_{di}$$

où , $B_i$ est la valeur de correction totale dans le $i^{\text{ème}}$ cycle cardiaque, et $T_{di}$ est $T_d$ dans le $i^{\text{ème}}$ cycle cardiaque,

lors du calcul de $T_s$ à l'étape S3, la valeur corrigée de la durée de propagation d'onde pulsatile associée à une

$$A_m = \frac{1}{8}\sum_{i=1}^{8} A_i \; , \quad T_{sm} = \frac{1}{8}\sum_{i=1}^{8} T_{si}$$

pression artérielle systolique $T_{sma}$ est calculée par : $T_{sma} = T_{sm}(1-A_m)$ ; où , $A_i$ est la valeur de correction totale dans le $i^{\text{ème}}$ cycle cardiaque, et $T_{si}$ est $T_s$ dans le $i^{\text{ème}}$ cycle cardiaque.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101229058 A
- CN 102811659 A
- CN 1127939 C
- US 5865755 A
- US 5857975 A
- US 5649543 A
- US 9364158 B
- EP 0413267 A